(19) Europäisches Patentamt
European Patent Office
Office européen des brevets

(11) EP 3 214 062 B1

(12) # EUROPEAN PATENT SPECIFICATION

(45) Date of publication and mention of the grant of the patent:
**08.01.2020 Bulletin 2020/02**

(21) Application number: **15854890.9**

(22) Date of filing: **02.11.2015**

(51) Int Cl.:
***C07C 45/82*** *(2006.01)* ***C07C 47/02*** *(2006.01)*

(86) International application number:
**PCT/KR2015/011651**

(87) International publication number:
**WO 2016/068676 (06.05.2016 Gazette 2016/18)**

(54) **DISTILLATION METHOD FOR THE SEPARATION OF N-BUTANAL AND ISO-BUTANAL**
DESTILLATIONSVERFAHREN ZUR TRENNUNG VON N-BUTANAL UND ISO-BUTANAL
PROCÉDÉ DE DISTILLATION POUR LA SÉPARATION DE N-BUTANAL ET ISO-BUTANAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.10.2014 KR 20140150416**

(43) Date of publication of application:
**06.09.2017 Bulletin 2017/36**

(73) Proprietor: **LG Chem, Ltd.**
**Seoul 07336 (KR)**

(72) Inventors:
- **LEE, Sung Kyu**
  **Daejeon 34122 (KR)**
- **SHIN, Joon Ho**
  **Daejeon 34122 (KR)**

(74) Representative: **Goddar, Heinz J. et al**
**Boehmert & Boehmert**
**Anwaltspartnerschaft mbB**
**Pettenkoferstrasse 22**
**80336 München (DE)**

(56) References cited:

| | |
|---|---|
| **JP-B2- 4 080 553** | **KR-A- 20010 105 400** |
| **KR-A- 20100 105 500** | **KR-B1- 960 004 888** |
| **RU-C1- 2 130 917** | **US-B1- 6 511 583** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 214 062 B1

## Description

[Technical Field]

The present application relates to a method distillation device for an isomer separation.

[Background Art]

This application is a National Stage Application of International Application No. PCT/KR2015/011651, filed November 2, 2015, and claims the benefit of Korean Patent Application No. 10-2014-0150416, filed October 31, 2014, and Korean Patent Application No. 10-2015-0153090, filed November 2, 2015.

Alkanols such as n-butanol are used in various applications such as, for example, a solvent for preparing a coating solution, in the chemical industry.

For example, n-butanol may be prepared through hydrogenation of n-butyl aldehyde. For example, butyl aldehyde may be prepared by allowing a gas mixture including propylene, carbon monoxide (CO), and hydrogen ($H_2$) to be subjected to oxo-reaction. In general, the prepared butyl aldehyde is a mixture of n-butyl aldehyde and iso-butyl aldehyde. N-butanol can be prepared by separating the n-butyl aldehyde from the mixture and allowing the separated n-butyl aldehyde to be subjected to hydrogenation.

In general cases, since a compound and an isomer thereof have a relatively small boiling point difference therebetween, compared to other compounds, it is difficult to separate the compound from the isomer thereof. For example, since a boiling point difference between n-butyl aldehyde and an isomer thereof, iso-butyl aldehyde, is very small, high energy is required to separate n-butyl aldehyde from iso-butyl aldehyde. Accordingly, considerable energy is consumed in obtaining high-purity n-butyl aldehyde and the purities of products may be decreased to reduce energy consumption in a separation process of the isomer.

US 6,511,583 discloses a two-step distillation of n-butyladehyde and isobutylaldehyde.

RU 2 130917 C1 discloses the two-step distillation of n-butylaldehyde and isobutylaldehyde.

[Disclosure]

[Technical Problem]

Therefore, the present invention has been made in view of the above problems, and it is an object of the present application to provide a distillation device for high purity isomer separation with energy efficient.

[Technical Solution]

The above problems are solved in accordance with the independent claims. Preferred embodiments result from the sub-claims.

The present application relates to a method using distillation device. In accordance with exemplary embodiments of the present application, the distillation device used in the inventive may increase separation efficiency of a process by minimizing energy loss occurring in a purification process of a mixture including an isomer, for example, a raw material including a compound represented by Formula 1 below and an isomer of the compound, and separation of high purity product. In particular, the distillation device of the present application provides optimized temperature and pressure for separation between n-butyl aldehyde and iso-butyl aldehyde using two distillation units, thus economically preparing high-purity n-butyl aldehyde.

Hereinafter, the distillation device used in the method of the present application will be described with reference to the accompanying drawings. The drawings are, however, provided as exemplary embodiments and the distillation device should not be understood as limited to the accompanying drawings.

FIG. 1 exemplarily illustrates a distillation device used in the method according to an embodiment of the present application. As illustrated in FIG. 1, in an exemplary embodiment, the distillation device includes two distillation units 10 and 20 and a heat exchanger 30. For example, the distillation device includes the first and second distillation units 10 and 20 and the heat exchanger 30. The first distillation unit 10 includes a first distillation column 100, a first condenser 101, a storage tank 102, and a first reboiler 103. The second distillation unit 20 includes a second distillation column 200, a second condenser 201, a storage tank 202, and a second reboiler 203.

The first and second distillation columns 100 and 200 are devices for various components separation included in a raw material using boiling point differences thereamong. In the distillation device used in the method of the present application, distillation columns with various shapes may be used, considering components of an introduced raw material or the boiling points of the components to be separated. A distillation column type which may be used in the distillation

device used in the method of the present application is not specifically limited and may be, for example, a distillation column with a general structure as illustrated in FIG. 1 or a dividing wall-type distillation column including a dividing wall therein. In an example, the interiors of the first and second distillation columns 100 and 200 may be divided into upper sections 110 and 210, lower sections 130 and 230, and intermediate sections 120 and 220, as illustrated in FIG. 1. The expression 「upper section」, as used in the present specification, means relatively upper portions of the first and second distillation columns 100 and 200. For example, when each of the first and second distillation columns 100 and 200 is tri-sectioned in a height or length direction thereof, the upper section may mean an uppermost section thereof. In addition, the expression 「lower section」 means a relatively lower portion of each of the first and second distillation columns 100 and 200. For example, when each of the first and second distillation columns 100 and 200 is tri-sectioned in a height or length direction thereof, the lower section may mean a lowest section thereof. In addition, when each of the first and second distillation columns 100 and 200 is tri-sectioned in a height or length direction thereof, the expression 「intermediate section」 as used in the present specification may mean an intermediate section among three divided sections and a section between the upper section 110 or 210 and the lower section 130 or 230 of each of the first and second distillation columns 100 and 200. In the present specification, the upper section, the lower section and the intermediate section of the distillation column are relative concepts. The tops of the first and second distillation columns 100 and 200 are included in the upper sections and the bottoms of the first and second distillation columns 100 and 200 are included in the lower sections thereof. Unless mentioned otherwise, the upper section has the same meaning as "the top of a column", and the lower section has the same meaning as "the bottom of a column". The first and second distillation columns 100 and 200 may be distillation columns with a theoretical plate number of 50 to 150, 70 to 140, or 90 to 130. The expression 「theoretical plate number」means the number of hypothetical areas or plates, in which two phases such as a vapor phase and a liquid phase establish an equilibrium with each other, of the first and second distillation columns 100 and 200.

In an embodiment, as illustrated in FIG. 1, the first distillation unit 10 includes the first distillation column 100 and the first condenser 101, the storage tank 102, and the first reboiler 103 respectively connected to the first distillation column 100. The second distillation unit 20 includes the second distillation column 200 and the second condenser 201, the storage tank 202, and the second reboiler 203 respectively connected to the second distillation column 200, as illustrated in FIG. 1. For example, the first distillation column 100, the first condenser 101, the storage tank 102, and the first reboiler 103 may be fluidically connected to each other such that fluid introduced into the first distillation column 100 flows thereinto. The second distillation column 200, the second condenser 201, the storage tank 202, and the second reboiler 203 may be fluidically connected to each other such that fluid introduced into the second distillation column 200 flows thereinto. The 「condenser」 is separately installed on the outside of the distillation column and performs cooling using, for example, a method of bringing a stream discharged from the top of the distillation column into contact with cooling water introduced from the outside. For example, the first condenser 101 of the first distillation column 100 may condense a first column top stream $F_{1-2}$ discharged from the upper section 110 of the first distillation column 100, and the second condenser 201 of the second distillation column 200 may condense a second column top stream $F_{2-2}$ discharged from the upper section 210 of the second distillation column 200. In addition, the 「reboiler」 means a heating device separately installed on the outside of the distillation column. Alternatively, the reboiler may be a device for re-heating and evaporating a stream including components with a high boiling point discharged from the bottom of the distillation column. For example, the first reboiler 103 of the first distillation column 100 may be a device for heating a column bottom stream $F_{1-3}$ discharged from the lower section 130 of the first distillation column 100, and the second reboiler 203 of the second distillation column 200 described below may be a device for heating a column bottom stream $F_{2-3}$ discharged from the lower section 230 of the second distillation column 200. The 「storage tank」 means a tank or a bath for temporarily storing a stream discharged from the distillation column and may be any tank or bath known in the art. For example, the first column top stream $F_{1-2}$ discharged from the upper section 110 of the first distillation column 100 is condensed in the first condenser 101, followed by being introduced into the storage tank 102 and stored therein. The second column top stream $F_{2-2}$ discharged from the upper section 210 of the second distillation column 200 may be condensed in the second condenser 201, followed by being introduced into the storage tank 202 and stored therein.

The first distillation column 100 includes a first supply port 121, and the second distillation column 200 includes a second supply port 221. In an embodiment, the first supply port 121 is located at the intermediate section 120 of the first distillation column 100, and the second supply port 221 is located at the intermediate section 220 of the second distillation column 200.

As illustrated in FIG. 1, the raw material including the compound represented by Formula 1 below and the isomer of the compound is introduced into the first supply port 121 of the first distillation column 100 and/or the second supply port 221 of the second distillation column 200:

[Formula 1]

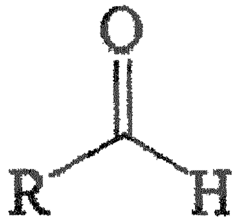

wherein R is a $C_1$ to $C_{12}$ alkyl group, for example, a $C_1$ to $C_{10}$ alkyl group, a $C_1$ to $C_8$ alkyl group, a $C_1$ to $C_6$ alkyl group, or a $C_1$ to $C_4$ alkyl group. In the methods of the invention the compound represented by Formula 1 is, n-butyl aldehyde, and the isomer of the compound is iso-butyl aldehyde.

For example, as illustrated in FIG. 1, in the case of the distillation device with a structure wherein the first distillation column 100 and the second distillation column 200 are connected in parallel (hereinafter also referred to as a「parallel structure」), the raw material including the compound represented by Formula 1 and the isomer of the compound is introduced into the first supply port 121 of the first distillation column 100 and the second supply port 221 of the second distillation column 200, respectively. When the distillation device used in the method of the present application includes the first distillation column 100 and the second distillation column 200 which are connected to each other in parallel, as illustrated in FIG. 1, energy reduction effects may be maximized.

In an example, the raw material introduced into the first supply port 121 of the first distillation column 100 is introduced into the intermediate section 120 of the first distillation column 100, and a raw material $F_{1-1}$ introduced into the intermediate section 120 of the first distillation column 100 is separately discharged into each of a column top stream discharged from the upper section 110 of the first distillation column 100 and a column bottom stream discharged from the lower section 130 of the first distillation column 100. In this case, the column bottom stream discharged from the lower section 130 of the first distillation column 100 may be separately discharged into at least one stream. For example, the raw material introduced into the first distillation column 100 may be separately discharged into each of the first column top stream $F_{1-2}$, and first, second and third column bottom streams $F_{1-3}$, $F_{1-4}$, and $F_{1-5}$ discharged from the lower section 130 of the first distillation column 100. The first column top stream $F_{1-2}$ discharged from the upper section 110 of the first distillation column 100 is introduced into the first condenser 101, a portion or all of the first column top stream $F_{1-2}$ passing through the first condenser 101 may be refluxed in the upper section 110 of the first distillation column 100 or stored as a product. In an example, a stream discharged from the first condenser 101 may be introduced into the storage tank 102 and stored therein, followed by being refluxed in the first distillation column 100 or stored as a product. In addition, the first column bottom stream $F_{1-3}$ discharged from the lower section 130 of the first distillation column 100 is introduced into the first reboiler 103. The first column bottom stream $F_{1-3}$ passing through the first reboiler 103 is introduced into the lower section 130 of the first distillation column 100 and the second column bottom stream $F_{1-4}$ discharged from the lower section 130 of the first distillation column 100 may be stored as a product. The first column bottom stream $F_{1-3}$ introduced into the first reboiler 103 may be heated by high-pressure steam passing through the first reboiler 103. The amount of this high-pressure steam may be properly controlled by the heat exchanger 30 described below. For example, when heat exchange in the heat exchanger 30 is sufficiently carried out, the high-pressure steam might not be used at all. However, when heat exchange is not smoothly carried out due to disturbance of a discharge rate of a raw material or a process, separation efficiency may be rapidly decreased. Accordingly, a proper amount of the high-pressure steam may be temporarily used such that robust separation efficiency can be maintained despite the disturbance.

As described above, in the case of a distillation device including the first distillation column 100 and the second distillation column 200 connected in parallel, a stream introduced into the second supply port 221 of the second distillation column 200 may include the raw material including the compound represented by Formula 1 and the isomer of the compound. The raw material introduced into the second supply port 221 of the second distillation column 200 is introduced into the intermediate section 220 of the second distillation column 200. A raw material $F_{2-1}$ introduced into the intermediate section 220 of the second distillation column 200 is separately discharged into a column top stream discharged from the upper section 210 of the second distillation column 200 and a column bottom stream discharged from the lower section 230 of the second distillation column 200. In this case, the column bottom stream discharged from the lower section 230 of the second distillation column 200 may be separately discharged into at least one stream. For example, the raw material introduced into the second distillation column 200 may be separately discharged into each of the second column top stream $F_{2-2}$ and a fourth column bottom stream $F_{2-3}$ and a fifth column bottom stream $F_{2-4}$ discharged from the lower section 230 of the second distillation column 200. The fourth column bottom stream $F_{2-3}$ discharged from the lower section 230 of the second distillation column 200 is introduced into the second reboiler 203. The fourth column bottom stream $F_{2-3}$ passing through the second reboiler 203 is introduced into the lower section 230 of the second distillation column 200, and the fifth column bottom stream $F_{2-4}$ discharged from the lower section 230 of the second

distillation column 200 may be stored as a product.

The third column bottom stream $F_{1-5}$ discharged from the lower section 130 of the first distillation column 100 and the second column top stream $F_{2-2}$ discharged from the upper section 210 of the second distillation column 200 are introduced into the heat exchanger 30. The 「heat exchanger」 is separately installed on the outside of the distillation column and performs heat exchange such that heat transfer between two fluid streams, the temperatures of which are different, is fluently carried out. For example, the heat exchanger 30 may allow heat exchange between the third column bottom stream $F_{1-5}$ discharged from the lower section 130 of the first distillation column 100 and the second column top stream $F_{2-2}$ discharged from the upper section 210 of the second distillation column 200. In the distillation device for use in the method of the present application, the third column bottom stream $F_{1-5}$ with a high boiling point discharged from the lower section 130 of the first distillation column 100 and the second column top stream $F_{2-2}$ with a low boiling point discharged from the upper section 210 of the second distillation column 200 exchange heat in the heat exchanger 30, thereby reducing energy required in condensation and heating processes in which the condenser or the reboiler is used.

The heat exchanger 30 may be directly or indirectly connected to pipes through which the third column bottom stream $F_{1-5}$ of the first distillation column 100 and the second column top stream $F_{2-2}$ of the second distillation column 200 pass. In an example, when the heat exchanger 30 is directly connected to the pipes through which the third column bottom stream $F_{1-5}$ of the first distillation column 100 and the second column top stream $F_{2-2}$ of the second distillation column 200 pass, heat exchange between the third column bottom stream $F_{1-5}$ and the second column top stream $F_{2-2}$ may be efficiently performed.

Heat exchange between the third column bottom stream $F_{1-5}$ and the second column top stream $F_{2-2}$ introduced into the heat exchanger 30 is carried out, the third column bottom stream $F_{1-5}$ passing through the heat exchanger 30 is refluxed in the lower section 130 of the first distillation column 100, the second column top stream $F_{2-2}$ passing through the heat exchanger 30 is introduced into the second condenser 201, and a portion or all of the second column top stream $F_{2-2}$ passing through the second condenser 201 may be refluxed in the upper section 210 of the second distillation column 200 or stored as a product. In an example, a stream discharged from the second condenser 201 is introduced in the storage tank 202 and stored therein. Subsequently, the stored stream may be refluxed in the second distillation column 200 or stored as a product.

In the heat exchanger 30, the third column bottom stream $F_{1-5}$ may be heat exchanged with the second column top stream $F_{2-2}$ before the third column bottom stream $F_{1-5}$ is refluxed in the first distillation column 100, and the second column top stream $F_{2-2}$ may exchange heat with the third column bottom stream $F_{1-5}$ before the second column top stream $F_{2-2}$ is introduced into the second condenser 201. For example, the second column top stream $F_{2-2}$ including a low boiling component discharged from the upper section 210 of the second distillation column 200 stops by the heat exchanger 30 before being refluxed in the upper section 210 of the second distillation column 200. At this time, heat is supplied to the heat exchanger 30. Accordingly, the second column top stream $F_{2-2}$ discharged from the second distillation column 200 may be refluxed, at a relatively low temperature, into the second distillation column 200. Accordingly, the quantity of heat neeeded for condensing the second column top stream $F_{2-2}$ discharged from the upper section 210 of the second distillation column 200 may be decreased, and costs necessary for the condensation process may be reduced by decreasing the amount of cooling water used in the condensation process in which the second condenser 201 is used. In addition, the third column bottom stream $F_{1-5}$, as a stream including a high boiling component discharged from the lower section 130 of the first distillation column 100 drops by the heat exchanger 30 before being refluxed in the lower section 130 of the first distillation column 100. At this time, heat transferred by the second column top stream $F_{2-2}$ may be supplied to the third column bottom stream $F_{1-5}$. Accordingly, the second column top stream $F_{2-2}$ supplies heat to the lower section 130 of the first distillation column 100 and thus the amount of steam used in the first reboiler 103 in order to heat the first column bottom stream $F_{1-3}$ discharged from the lower section 130 of the first distillation column 100 is reduced, thereby reducing costs.

Hereinafter, a separation process between n-butyl aldehyde and iso-butyl aldehyde, as an isomer thereof, using a distillation device according to an embodiment of the present application will be described in detail.

In an example, the raw material $F_{1-1}$ including n-butyl aldehyde and iso-butyl aldehyde, as an isomer thereof, is introduced into each of the first supply port 121 of the first distillation column 100 and the second supply port 221 of the second distillation column 200.

In this case, a stream including a large amount of iso-butyl aldehyde with a relatively low boiling point among components that are included in the raw material $F_{1-1}$ introduced into the first supply port 121 may be discharged as the first column top stream $F_{1-2}$ from the upper section 110 of the first distillation column 100, and a stream including a large amount of n-butyl aldehyde with a relatively high boiling point may be discharged as the first, second and third column bottom streams $F_{1-3}$, $F_{1-4}$, and $F_{1-5}$ from the lower section 130 of the first distillation column 100. The first column top stream $F_{1-2}$ discharged from the upper section 110 of the first distillation column 100 is introduced into the storage tank 102 via the first condenser 101. A portion of the stream discharged from the storage tank 102 is refluxed in the upper section 110 of the first distillation column 100, and a portion of the remainder of the stream may be stored as a product.

The product may be high-purity iso-butyl aldehyde. Meanwhile, the first column bottom stream $F_{1-3}$ discharged from the lower section 130 of the first distillation column 100 may be refluxed in the lower section 130 of the first distillation column 100 via the first reboiler 103, and the second column bottom stream $F_{1-4}$ may be stored as a product. The product may be high-purity n-butyl aldehyde. In addition, the third column bottom stream $F_{1-5}$ may exchange heat with the second column top stream $F_{2-2}$ of the second distillation column 200 in the heat exchanger 30, followed by being refluxed in the lower section 130 of the first distillation column 100.

In addition, a stream including a large amount of iso-butyl aldehyde with a relatively low boiling point among components included in the raw material stream $F_{2-1}$ introduced into the second supply port 221 may be discharged as the second column top stream $F_{2-2}$ from the upper section 210 of the second distillation column 200, and a stream including a large amount of n-butyl aldehyde with a relatively high boiling point among the components may be discharged as the fourth and fifth column bottom streams $F_{2-3}$ and $F_{2-4}$ from the lower section 230 of the second distillation column 200. The discharged second column top stream $F_{2-2}$ exchanges heat with the third column bottom stream $F_{1-5}$ of the first distillation column 100 in the heat exchanger 30 and is then introduced into the storage tank 202 via the second condenser 201. A portion of the stream discharged from the storage tank 202 may be refluxed in the upper section 210 of the second distillation column 200, and another portion of the remainder of the stream may be stored as a product. The product may be high-purity iso-butyl aldehyde. In addition, a stream including a component with a relatively high boiling point among components included in the raw material $F_{2-1}$ may be discharged as the fourth and fifth column bottom streams $F_{2-3}$ and $F_{2-4}$ from the lower section 230 of the second distillation column 200. The fourth column bottom stream $F_{2-3}$ may be refluxed in the lower section 230 of the second distillation column 200 via the second reboiler 203 and the fifth column bottom stream $F_{2-4}$ may be stored as a product. The product may be high-purity n-butyl aldehyde.

In the present specification, the expression「stream including a low boiling component」means a stream including a large amount of a component with a relatively low boiling point in a raw material stream including a low boiling component and a high boiling component. For example, the stream including a low boiling component means streams discharged from the upper sections 110 and 210 of the first and second distillation columns 100 and 200. In addition, the expression「stream including a high boiling component」means a stream including a large amount of a component with a relatively high boiling point in a raw material stream including a low boiling component and a high boiling component. For example, the stream including a high boiling component means a stream including a large amount of a component with a relatively high boiling point discharged from the lower sections 130 and 230 of the first and second distillation columns 100 and 200. The expression「stream including a large amount of a component」means a stream wherein the content of each of a low boiling component, which is included in a stream discharged from the upper sections 110 and 210 of the first and second distillation columns 100 and 200, and a high boiling component, which is included in stream discharged from the lower sections 130 and 230 of the first and second distillation columns 100 and 200, is higher than the content of each of the low boiling component and the high boiling component included in the raw material $F_{1-1}$. For example, the content of each of the low boiling component included in the first column top stream $F_{1-2}$ of the first distillation column 100 and the low boiling component included in the second column top stream $F_{2-2}$ of the second distillation column 200 may be 50% by weight or more, 80% by weight or more, 90% by weight or more, 95% by weight or more or 99% by weight or more. Alternatively, the contents of a high boiling component included in each of the first column top stream $F_{1-2}$, and the first, second and third column bottom streams $F_{1-3}$, $F_{1-4}$, and $F_{1-5}$ of the first distillation column 100 and a high boiling component included in each of the fourth and fifth column bottom streams $F_{2-3}$ and $F_{2-4}$ of the second distillation column 200 may respectively be 50% by weight or more, 80% by weight or more, 90% by weight or more, 95% by weight or more, or 99% by weight or more.

FIG. 2 exemplarily illustrates a distillation device for use in a method according to another embodiment of the present application.

As illustrated in FIG. 2, when a distillation device includes the first distillation column 100 and the second distillation column 200 connected in series (hereinafter also referred to as a「serial structure」), the raw material including the compound represented by Formula 1 and the isomer of the compound is introduced into the first supply port 121 of the first distillation column 100. In this case, the second column bottom stream $F_{1-4}$ of the first distillation column 100 is introduced into the second supply port 221 of the second distillation column 200. As illustrated in FIG. 2, when the distillation device for use in the method of the present application includes the first distillation column 100 and the second distillation column 200 connected in series, the purity of prepared n-butyl aldehyde may be maximized.

In an example, as illustrated in FIG. 2, the raw material introduced into the first supply port 121 of the first distillation column 100 is introduced into the intermediate section 120 of the first distillation column 100, and the raw material $F_{1-1}$ introduced into the intermediate section 120 of the first distillation column 100 is separately discharged into each of a column top stream discharged from the upper section 110 of the first distillation column 100 and a column bottom stream discharged from the lower section 130 of the first distillation column 100. In this case, as in the aforementioned distillation device with a parallel structure, the column bottom stream discharged from the lower section 130 of the first distillation column 100 may be separately discharged into at least one stream. For example, the raw material introduced into the first distillation column 100 may be separately discharged into each of the first column top stream

$F_{1-2}$, and the first, second and third column bottom streams $F_{1-3}$, $F_{1-4}$, and $F_{1-5}$ discharged from the lower section 130 of the first distillation column 100. The first column top stream $F_{1-2}$ discharged from the upper section 110 of the first distillation column 100 is introduced into the first condenser 101, a portion or all of the first column top stream $F_{1-2}$ passing through the first condenser 101 is refluxed in the upper section 110 of the first distillation column 100 or may be stored as a product. In an example, a stream discharged from the first condenser 101 may be introduced into the storage tank 102 and stored therein, followed by being refluxed in the first distillation column 100 or stored as a product. In addition, the first column bottom stream $F_{1-3}$ discharged from the lower section 130 of the first distillation column 100 is introduced into the first reboiler 103. The first column bottom stream $F_{1-3}$ passing through the first reboiler 103 may be introduced into the lower section 130 of the first distillation column 100.

As described above, in the case of a distillation device including the first distillation column 100 and the second distillation column 200 connected in series, a stream introduced into the second supply port 221 of the second distillation column 200 may be the second column bottom stream $F_{1-4}$ of the first distillation column 100. The second column bottom stream $F_{1-4}$ introduced into the second supply port 221 of the second distillation column 200 is introduced into the intermediate section 220 of the second distillation column 200. The second column bottom stream $F_{1-4}$ introduced into the intermediate section 220 of the second distillation column 200 is separately discharged into each of a column top stream discharged from the upper section 210 of the second distillation column 200 and a column bottom stream discharged from the lower section 230 of the second distillation column 200. In this case, as in the aforementioned distillation device with a parallel structure, the column bottom stream discharged from the lower section 230 of the second distillation column 200 may be separately discharged into at least one stream. For example, the stream introduced into the second distillation column 200 may be separately discharged into each of the second column top stream $F_{2-2}$ and the fourth column bottom stream $F_{2-3}$ and the fifth column bottom stream $F_{2-4}$ discharged from the lower section 230 of the second distillation column 200. The fourth column bottom stream $F_{2-3}$ discharged from the lower section 230 of the second distillation column 200 is introduced into the second reboiler 203. The fourth column bottom stream $F_{2-3}$ passing through the second reboiler 203 is introduced into the lower section 230 of the second distillation column 200, and the fifth column bottom stream $F_{2-4}$ discharged from the lower section 230 of the second distillation column 200 may be stored as a product.

The third column bottom stream $F_{1-5}$ discharged from the lower section 130 of the first distillation column 100 and the second column top stream $F_{2-2}$ discharged from the upper section 210 of the second distillation column 200 are introduced into the heat exchanger 30. As described above, the heat exchanger 30 may allow heat exchange between the third column bottom stream $F_{1-5}$ discharged from the lower section 130 of the first distillation column 100 and the second column top stream $F_{2-2}$ discharged from the upper section 210 of the second distillation column 200. In the distillation device for use in the method of the present application, the third column bottom stream $F_{1-5}$ with a high boiling point discharged from the lower section 130 of the first distillation column 100 and the second column top stream $F_{2-2}$ with a low boiling point discharged from the upper section 210 of the second distillation column 200 are heat-exchanged between each other in the heat exchanger 30, thereby reducing energy required in condensation and heating processes, in which the condenser or the reboiler is used, and preparing high-purity n-butyl aldehyde.

The heat exchanger 30 is the same as that for the aforementioned distillation device including the first distillation column 100 and the second distillation column 200 which are connected to each other in parallel, and description thereof is thus omitted.

Hereinafter, a process of separating n-butyl aldehyde and iso-butyl aldehyde, as an isomer thereof, will be described in more detail using a distillation device including the first distillation column 100 and the second distillation column 200, which are connected in series, according to another embodiment of the present application.

In an example, the raw material $F_{1-1}$ including n-butyl aldehyde and iso-butyl aldehyde, as an isomer thereof is introduced into the first supply port 121 of the first distillation column 100.

In this case, a stream including a large amount of iso-butyl aldehyde with a relatively low boiling point among components that are included in the raw material $F_{1-1}$ introduced into the first supply port 121 may be discharged as the first column top stream $F_{1-2}$ from the upper section 110 of the first distillation column 100, and a stream including a large amount of n-butyl aldehyde with a relatively high boiling point may be discharged as the first, second and third column bottom streams $F_{1-3}$, $F_{1-4}$, and $F_{1-5}$ from the lower section 130 of the first distillation column 100. The first column top stream $F_{1-2}$ discharged from the upper section 110 of the first distillation column 100 is introduced into the storage tank 102 via the first condenser 101. A portion of a stream discharged from the storage tank 102 is refluxed in the upper section 110 of the first distillation column 100, and a portion of the remainder of the stream may be stored as a product. The product may be high purity iso-butyl aldehyde. Meanwhile, the first column bottom stream $F_{1-3}$ discharged from the lower section 130 of the first distillation column 100 may be refluxed in the lower section 130 of the first distillation column 100 via the first reboiler 103, and the second column bottom stream $F_{1-4}$ may be introduced into the second supply port 221 of the second distillation column 200. In addition, the third column bottom stream $F_{1-5}$ may exchange heat with the second column top stream $F_{2-2}$ of the second distillation column 200 in the heat exchanger 30, followed by being refluxed in the lower section 130 of the first distillation column 100.

In addition, the second column bottom stream $F_{1-4}$ introduced into the second supply port 221 includes n-butyl aldehyde and a high boiling component. Accordingly, a stream including a large amount of n-butyl aldehyde with a relatively low boiling point among components included in the second column bottom stream $F_{1-4}$ may be discharged as the second column top stream $F_{2-2}$ from the upper section 210 of the second distillation column 200, and a stream including components with relatively high boiling points may be discharged as the fourth and fifth column bottom streams $F_{2-3}$ and $F_{2-4}$ from the lower section 230 of the second distillation column 200. The discharged second column top stream $F_{2-2}$ exchanges heat with the third column bottom stream $F_{1-5}$ of the first distillation column 100 in the heat exchanger 30 and is then introduced into the storage tank 202 via the second condenser 201. A portion of the stream discharged from the storage tank 202 may be refluxed in the upper section 210 of the second distillation column 200, and a portion of the remainder of the stream may be stored as a product. The product may be ultrahigh-purity n-butyl aldehyde. In addition, a stream including a component with a relatively high boiling point among components included in the second column top stream $F_{2-2}$ may be discharged as the fourth and fifth column bottom streams $F_{2-3}$ and $F_{2-4}$ from the lower section 230 of the second distillation column 200. The fourth column bottom stream $F_{2-3}$ may be refluxed in the lower section 230 of the second distillation column 200 via the second reboiler 203 and the fifth column bottom stream $F_{2-4}$ may be stored as a product. The product may include, for example, n-butyl aldehyde, butyl alcohol, or dimers thereof, and trimers thereof.

In an example, a portion of the fifth column bottom stream $F_{2-4}$ discharged from the lower section 230 of the second distillation column 200 may be introduced into the lower section 130 of the first distillation column 100, for example, a 45th to 145th plate of the first distillation column 100 with a theoretical plate number of 50 to 150. Accordingly, n-butyl aldehyde that may remain in the fifth column bottom stream $F_{2-4}$ may be supplied to the lower section 130 of the first distillation column 100, thereby preparing n-butyl aldehyde with higher purity. In this case, a ratio of a discharge rate (ton/hr) of the stream introduced into the lower section 130 of the first distillation column 100 to a discharge rate (ton/hr) of the fifth column bottom stream $F_{2-4}$ discharged from the lower section 230 of the second distillation column 200 may be 1:0.85 to 1:0.95. By controlling the flow ratio of the stream introduced into the lower section 130 of the first distillation column 100 within this range, n-butyl aldehyde with higher purity may be prepared.

The distillation device for use in the method of the present application satisfies Equation 1 below.

[Equation 1]

$$T_{t-2} - T_{b-3} \geq 8^\circ C$$

wherein $T_{t-2}$ indicates a temperature of the second column top stream $F_{2-2}$, and $T_{b-3}$ indicates a temperature of the third column bottom stream $F_{1-5}$.

When the distillation device satisfies Equation 1, the compound represented by Formula 1, particularly n-butyl aldehyde, may be separated in superior efficiency and high purity using the distillation device with the aforementioned parallel structure or serial structure. That is, by controlling the distillation device such that a temperature difference between the second column top stream $F_{2-2}$ and the third column bottom stream $F_{1-5}$ satisfies Equation 1, heat exchange efficiency between the second column top stream $F_{2-2}$ and the third column bottom stream $F_{1-5}$ may be maximized. Accordingly, the compound represented by Formula 1, particularly n-butyl aldehyde, may be separated in superior efficiency and high purity.

In an example, so long as a temperature difference between the second column top stream $F_{2-2}$ discharged from the upper section 210 of the second distillation column 200 and the third column bottom stream $F_{1-5}$ discharged from the lower section 130 of the first distillation column 100 satisfies Equation 1, there is no specific limitation. For example, the temperature difference may be 8°C or more, 9°C or more, 10°C or more, or 13°C or more. Since heat exchange efficiency is superior with increasing temperature difference between the second column top stream $F_{2-2}$ discharged from the upper section 210 of the second distillation column 200 and the third column bottom stream $F_{1-5}$ discharged from the lower section 130 of the first distillation column 100, the maximum value of the temperature difference is not specifically limited. For example, a temperature difference between the second column top stream $F_{2-2}$ discharged from the upper section 210 of the second distillation column 200 and the third column bottom stream $F_{1-5}$ discharged from the lower section 130 of the first distillation column 100 may be 100°C or less, considering process efficiency.

The distillation device of the present application satisfies Equation 2 below:

[Equation 2]

$$P_2/P_1 \geq 20,$$

wherein $P_1$ indicates a pressure ($kg/cm^2g$) of the upper section 110 of the first distillation column 100, and $P_2$ indicates a pressure ($kg/cm^2g$) of the upper section 210 of the second distillation column 200.

When the distillation device satisfies Equation 2, the compound represented by Formula 1, particularly n-butyl aldehyde, may be separated in superior efficiency and high purity using the distillation device with the aforementioned parallel structure or serial structure. That is, by controlling the distillation device such that a ratio of the pressure of the upper section 210 of the second distillation column 200 to the pressure of the upper section 110 of the first distillation column 100 satisfies Equation 2, heat exchange efficiency between the second column top stream $F_{2-2}$ and the third column bottom stream $F_{1-5}$ may be maximized. Accordingly, the compound represented by Formula 1, particularly n-butyl aldehyde, may be separated in superior efficiency and high purity.

For example, in order to increase the heat exchange efficiency of the heat exchanger 30, the interior temperature of the first distillation column 100 may be kept lower than the interior temperature of the second distillation column 200, and thus, the pressure of the upper section 110 of the first distillation column 100 may be kept lower than that of the upper section 210 of the second distillation column 200.

In an example, so long as a ratio of the pressure of the upper section 210 of the second distillation column 200 to the pressure of the upper section 110 of the first distillation column 100 satisfies Equation 2, there is no specific limitation. For example, the ratio may be 20 or more, 25 or more, 35 or more, 50 or more, 80 or more, or 120 or more. Since heat exchange efficiency improves with increasing ratio of the pressure of the upper section 210 of the second distillation column 200 to the pressure of the upper section 110 of the first distillation column 100, the maximum value of the ratio is not specifically limited. For example, the ratio of the pressure of the upper section 210 of the second distillation column 200 to the pressure of the upper section 110 of the first distillation column 100 may be 300 or less, or 200 or less, considering process efficiency.

When the distillation device for use in the method of the present application has the aforementioned parallel structure, the temperature of the second column top stream $F_{2-2}$ discharged from the upper section 210 of the second distillation column 200 is not specifically limited so long as Equation 1 is satisfied. The temperature may be 100 to 110°C, for example, 102°C to 108°C or 104°C to 106°C. In addition, the temperature of the third column bottom stream $F_{1-5}$ discharged from the lower section 130 of the first distillation column 100 is not specifically limited so long as Equation 1 is satisfied. The temperature may be 90°C to 100°C, for example, 92°C to 98°C or 94°C to 96°C. In this case, the pressure of the upper section 110 of the first distillation column 100 is not specifically limited so long as Equation 2 is satisfied. The pressure may be 0.01 to 0.1 $Kg/cm^2g$, 0.01 to 0.07 $Kg/cm^2g$, or 0.015 to 0.03 $Kg/cm^2g$. In addition, the pressure of the upper section 210 of the second distillation column 200 is not specifically limited so long as Equation 2 is satisfied. The pressure may be 2.3 to 2.7 $Kg/cm^2g$, 2.35 to 2.65 $Kg/cm^2g$, or 2.4 to 2.6 $Kg/cm^2g$.

In an example, when the distillation device for use in the method of the present application has the aforementioned parallel structure, the temperature of the upper section 110 of the first distillation column 100 may be 60°C to 70°C, for example, 62°C to 68°C or 64°C to 66°C, and the temperature of the lower section 130 of the first distillation column 100 may be 90°C to 100°C, for example, 92°C to 98°C or 94°C to 96°C, but the present application is not limited thereto. In this case, the temperature of the upper section 210 of the second distillation column 200 may be 100 to 110°C, for example, 102°C to 108°C or 104°C to 106°C, and the temperature of the lower section 230 of the second distillation column 200 may be 120 to 140°C, for example, 124°C to 138°C or 126°C to 134°C, but the present application is not limited thereto.

In an example, when the distillation device for use in the method of the present application has the aforementioned parallel structure, Equation 3 below may be satisfied.

[Equation 3]

$$0.3 \leq F_1/F_2 \leq 3.0$$

wherein $F_1$ indicates a discharge rate of a raw material (ton/hr) introduced into the first supply port 121 of the first distillation column 100, and $F_2$ indicates a discharge rate of a raw material (ton/hr) introduced into the second supply port 221 of the second distillation column 200.

In the distillation device, energy reduction effect may be maximized by controlling a ratio of the discharge rate of the raw material $F_{1-1}$ introduced into the first supply port 121 of the first distillation column 100 to the discharge rate of the raw material $F_{2-1}$ introduced into the second supply port 221 of the second distillation column 200 within the range of Equation 3.

In an example, the ratio of the discharge rate of the raw material $F_{1-1}$ introduced into the first supply port 121 of the first distillation column 100 to the discharge rate of the raw material $F_{2-1}$ introduced into the second supply port 221 of the second distillation column 200 is not specifically limited so long as the ratio is within the aforementioned range. For example, the ratio may be 0.3 to 3.0, 0.6 to 2.0, 0.7 to 1.7. 0.8 to 1.4, or 0.9 to 1.2.

In addition, the discharge rate of the raw material $F_{1-1}$ introduced into the first supply port 121 of the first distillation column 100 is not specifically limited so long as Equation 3 is satisfied, and may be 10 to 30 ton/hr, for example, 14 to 26 ton/hr or 18 to 22 ton/hr. The discharge rate of the raw material $F_{2-1}$ introduced into the second supply port 221 of the second distillation column 200 is not specifically limited so long as Equation 3 is satisfied, and may be 10 to 30 ton/hr, for example, 14 to 26 ton/hr or 18 to 22 ton/hr.

When the distillation device for use in the method of the present application has the aforementioned parallel structure, the content of iso-butyl aldehyde in each of the first column top stream $F_{1-2}$ discharged from the upper section 110 of the first distillation column 100 and the second column top stream $F_{2-2}$ discharged from the upper section 210 of the second distillation column 200 may be 90% or more, preferably 99% or more. The content of n-butyl aldehyde in each of the second column bottom stream $F_{1-4}$ discharged from the lower section 130 of the first distillation column 100 and the fifth column bottom stream $F_{2-4}$ discharged from the lower section 230 of the second distillation column 200 may be 90% or more, preferably 99% or more.

When the distillation device for use in the method of the present application has the aforementioned serial structure, the temperature of the second column top stream $F_{2-2}$ discharged from the upper section 210 of the second distillation column 200 is not specifically limited so long as Equation 1 is satisfied, and may be 100 to 110°C, for example, 102°C to 108°C or 104°C to 106°C. In addition, the temperature of the third column bottom stream $F_{1-5}$ discharged from the lower section 130 of the first distillation column 100 is not specifically limited so long as Equation 1 is satisfied, and may be 90°C to 100°C, for example, 92°C to 98°C or 94°C to 96°C. In this case, the pressure of the upper section 110 of the first distillation column 100 is not specifically limited so long as Equation 2 is satisfied, and may be 0.01 to 0.1 $Kg/cm^2g$, 0.012 to 0.07 $Kg/cm^2g$, or 0.015 to 0.03 $Kg/cm^2g$. In addition, the pressure of the upper section 210 of the second distillation column 200 is not specifically limited so long as Equation 2 is satisfied, and may be 1.0 to 2.0 $Kg/cm^2g$, 1.2 to 2.0 $Kg/cm^2g$, or 1.4 to 1.6 $Kg/cm^2g$.

In an example, when the distillation device for use in the method of the present application has the aforementioned serial structure, the temperature of the upper section 110 of the first distillation column 100 may be 60°C to 70°C, for example, 62°C to 68°C or 64°C to 66°C, and the temperature of the lower section 130 of the first distillation column 100 may be 90°C to 100°C, for example, 92°C to 98°C or 94°C to 96°C, but the present application is not limited thereto. In this case, the temperature of the upper section 210 of the second distillation column 200 may be 100 to 110°C, for example, 102°C to 108°C or 104°C to 106°C, and the temperature of the lower section 230 of the second distillation column 200 may be 120 to 140°C, for example, 124°C to 138°C or 126°C to 134°C, but the present application is not limited thereto.

When the distillation device for use in the method of the present application has the aforementioned serial structure, the content of iso-butyl aldehyde in the first column top stream $F_{1-2}$ discharged from the upper section 110 of the first distillation column 100 may be 90% or more, preferably 99% or more, and the content of n-butyl aldehyde in the second column top stream $F_{2-2}$ discharged from the upper section 210 of the second distillation column 200 may be 90% or more, preferably 99% or more.

The present application also relates to a method of preparing the compound represented by Formula 1.

The preparation method according to an exemplary embodiment of the present application may be carried out using the aforementioned distillation device, and thus, the same contents as the descriptions of the aforementioned distillation device are omitted.

In an embodiment, the preparation method of the present application includes i) a step of introducing the raw material including the compound represented by Formula 1 below and the isomer of the compound into each of the first supply port 121 of the first distillation column 100 and the second supply port 221 of the second distillation column 200; ii) a step of discharging the raw material introduced into the first supply port 121 to each of the first column top stream $F_{1-2}$ discharged from the upper section 110 of the first distillation column 100; and the first column top stream $F_{1-2}$, and the first, second and third column bottom streams $F_{1-3}$, $F_{1-4}$, and $F_{1-5}$ discharged from the lower section 130 of the first distillation column 100; iii) a step of discharging the raw material introduced into the second supply port 221 to each of the second column top stream $F_{2-2}$ discharged from the upper section 210 of the second distillation column 200; and the fourth and fifth column bottom streams $F_{2-3}$ and $F_{2-4}$ discharged from the lower section 230 of the second distillation column 200; iv) a step of exchanging heat between the second column top stream $F_{2-2}$ and the third column bottom stream $F_{1-5}$; and v) a step of separating the compound represented by Formula 1 from the lower section 130 of the first distillation column 100, and the isomer of the compound represented by Formula 1 from the upper section 110 of the first distillation column 100 and the upper section 210 of the second distillation column 200:

[Formula 1]

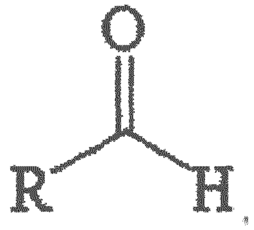

wherein R is a $C_1$ to $C_{12}$ alkyl group. The compound represented by Formula 1 is n-butyl aldehyde.

The preparation method may be carried out using a distillation device with the aforementioned parallel structure. The distillation device with the parallel structure is the same as those described above, description thereof thus being omitted.

Steps i) to v) are each independently, organically connected, and thus, boundaries therebetween are not clearly divided according to chronological order. Each of steps i) to v) may be carried out sequentially or independently at the same time.

The preparation method satisfies Equations 1 and 2 below. Descriptions therefor are the same as those described above, thus being omitted.

[Equation 1]

$$T_{t-2} - T_{b-3} \geq 8℃$$

[Equation 2]

$$P_2/P_1 \geq 20$$

wherein $T_{t-2}$ indicates a temperature of the second column top stream $F_{2-2}$, and $T_{b-3}$ indicates a temperature of the third column bottom stream $F_{1-5}$, and

$P_1$ indicates a pressure (kg/cm$^2$g) of the upper section 110 of the first distillation column 100, and $P_2$ indicates a pressure (kg/cm$^2$g) of the upper section 210 of the second distillation column 200.

In another embodiment, the preparation method of the present application includes a) a step of introducing the raw material including the compound represented by Formula 1 below and the isomer of the compound into the first supply port 121 of the first distillation column 100; b) a step of discharging the introduced raw material to each of the first column top stream $F_{1-2}$ discharged from the upper section 110 of the first distillation column 100; and the first column top stream $F_{1-2}$, and the first, second and third column bottom streams $F_{1-3}$, $F_{1-4}$, and $F_{1-5}$ discharged from the lower section 130 of the first distillation column 100; c) a step of introducing the first column bottom stream $F_{1-3}$ into the second supply port 221 of the second distillation column 200; d) a step of discharging the stream introduced into the second supply port 221 to each of the second column top stream $F_{2-2}$ discharged from the upper section 210 of the second distillation column 200; and the fourth and fifth column bottom streams $F_{2-3}$ and $F_{2-4}$ discharged from the lower section 230 of the second distillation column 200; e) a step of exchanging heat between the second column top stream $F_{2-2}$ and the third column bottom stream $F_{1-5}$; and f) a step of separating the compound represented by Formula 1 from the upper section 210 of the second distillation column 200, and the isomer of the compound from the upper section 110 of the first distillation column 100:

[Formula 1]

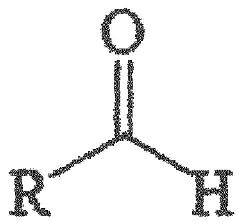

wherein R is a $C_1$ to $C_{12}$ an alkyl group.

The preparation method may be carried out using a distillation device with the aforementioned serial structure. The distillation device with the serial structure is the same as those described above, description thereof thus being omitted.

As described above, steps a) to f) are each independently, organically connected, and thus, boundaries therebetween are not clearly divided according to chronological order. Each of steps a) to f) may be carried out sequentially or independently at the same time.

The preparation method satisfies Equations 1 and 2 below. Descriptions thereof are the same as those described above, thus being omitted:

[Equation 1]

$$T_{t-2} - T_{b-3} \geq 8\,℃$$

[Equation 2]

$$P_2/P_1 \geq 20$$

wherein $T_{t-2}$ indicates a temperature of the second column top stream and $T_{b-3}$ indicates a temperature of the third column bottom stream $F_{1-5}$, and

$P_1$ indicates a pressure (kg/cm$^2$g) of the upper section 110 of the first distillation column 100 and $P_2$ indicates a pressure (kg/cm$^2$g) of the upper section 210 of the second distillation column 200.

[Advantageous Effects]

A method using a distillation device of the present application can minimize energy loss occurring during a purification process of a mixture including an isomer, namely, a raw material including n-butyl aldehyde and iso-butyl aldehyde, and can increase separation efficiency by obtaining a high-purity product.

[Description of Drawings]

FIG. 1 exemplarily illustrates a distillation device for use in the method according to an embodiment of the present application.

FIG. 2 exemplarily illustrates a distillation device for use in the method according to another embodiment of the present application.

FIG. 3 exemplarily illustrates a general separation device used in a comparative example.

[Best Mode]

Now, the present invention will be described in more detail with reference to examples according to the present invention and comparative examples not according to the present invention. These examples are provided for illustrative purposes only and should not be construed as limiting the scope and spirit of the present invention.

**Example 1**

N-butyl aldehyde and iso-butyl aldehyde were separated by means of a distillation device illustrated in FIG. 1. In particular, a raw material including n-butyl aldehyde and iso-butyl aldehyde was introduced into each of a first distillation column with a theoretical plate number of 100 and a second distillation column with a theoretical plate number of 100. In this case, a ratio of the discharge rate of the raw material introduced into the first distillation column to the discharge rate of the raw material introduced into the second distillation column was controlled to 2:3.

A portion of a first column top stream discharged from an upper section of the first distillation column was refluxed in the upper section of the first distillation column via a first condenser. A portion of the remainder of the first column top stream was separated as a product including iso-butyl aldehyde and stored. A portion of a first column bottom stream discharged from a lower section of the first distillation column was refluxed in the lower section of the first distillation column via a first reboiler. A second column bottom stream discharged from the lower section of the first distillation column was separated as a product including n-butyl aldehyde and stored. A third column bottom stream discharged from the lower section of the first distillation column was introduced into a heat exchanger and heat-exchanged with a second column top stream of the second distillation column introduced into the heat exchanger, followed by being refluxed in the lower section of the first distillation column via the heat exchanger. In this case, operation pressure of the upper section of the first distillation column was adjusted to 0.02 $Kg/cm^2g$ and operation temperature thereof was adjusted to 65°C. Operation temperature of the lower section of the first distillation column was adjusted to 95°C.

Meanwhile, the second column top stream discharged from an upper section of the second distillation column was introduced into the heat exchanger and heat-exchanged with the third column bottom stream. Subsequently, a portion of the second column top stream having passed through the heat exchanger and a second condenser was refluxed in the upper section of the second distillation column, and a portion of the remainder of the second column top stream was separated as a product including iso-butyl aldehyde. A fourth column bottom stream discharged from a lower section of the second distillation column was refluxed in the lower section of the second distillation column via a second reboiler, and a fifth column bottom stream discharged from the lower section of the second distillation column was separated as a product including n-butyl aldehyde. In this case, operation pressure of the upper section of the second distillation column was adjusted to 2.5 $Kg/cm^2g$, and operation temperature thereof was adjusted to 105°C. Operation temperature of the lower section of the second distillation column was adjusted to 129°C.

N-butyl aldehyde and iso-butyl aldehyde were separated by means of the distillation device of Example 1. With regard to this, used energy amount, recovery amount, reduction amount, reduction rate, and the purity of an n-butyl aldehyde/iso-butyl aldehyde product are summarized in Table 1 below.

**Example 2**

N-butyl aldehyde and iso-butyl aldehyde were separated in the same manner as in Example 1, except that the operation conditions of the first and second distillation columns were changed as disclosed in Table 1 below.

N-butyl aldehyde and iso-butyl aldehyde were separated by means of a distillation device of Example 2. With regard to this, used energy amount, recovery amount, reduction amount, reduction rate, and the purity of an n-butyl aldehyde/iso-butyl aldehyde product are summarized in Table 1 below.

**Example 3**

N-butyl aldehyde and iso-butyl aldehyde were separated in the same manner as in Example 1, except that the operation conditions of the first and second distillation columns were changed as disclosed in Table 1 below.

N-butyl aldehyde and iso-butyl aldehyde were separated by means of a distillation device of Example 3. With regard to this, used energy amount, recovery amount, reduction amount, reduction rate, and the purity of an n-butyl aldehyde/iso-butyl aldehyde product are summarized in Table 1 below.

**Example 4**

N-butyl aldehyde and iso-butyl aldehyde were separated in the same manner as in Example 2, except that the operation conditions of the first and second distillation columns were changed as disclosed in Table 1 below.

N-butyl aldehyde and iso-butyl aldehyde were separated by means of a distillation device of Example 4. With regard to this, used energy amount, recovery amount, reduction amount, reduction rate, and the purity of an n-butyl aldehyde/iso-butyl aldehyde product are summarized in Table 1 below.

**Example 5**

N-butyl aldehyde and iso-butyl aldehyde were separated in the same manner as in Example 2, except that the operation conditions of the first and second distillation columns were changed as disclosed in Table 1 below.
N-butyl aldehyde and iso-butyl aldehyde were separated by means of a distillation device of Example 5. With regard to this, used energy amount, recovery amount, reduction amount, reduction rate, and the purity of an n-butyl aldehyde/iso-butyl aldehyde product are summarized in Table 1 below.

**Comparative Example 1**

As illustrated in FIG. 3, n-butyl aldehyde and iso-butyl aldehyde were separated by means of one distillation column. A portion of a stream with a low boiling point discharged from an upper section of the distillation column was refluxed in the distillation column via a condenser, and a portion of the remainder of the stream was produced as a product including iso-butyl aldehyde. A portion of a stream discharged from a lower section of the distillation column was refluxed in the distillation column via a reboiler, and a portion of the remainder of the stream was separated as a product including n-butyl aldehyde. In this case, operation pressure of the upper section of the distillation column was adjusted to 0.32 $Kg/cm^2g$, and the operation temperature thereof was adjusted to 73°C. Operation temperature of the lower section of the distillation column was adjusted to 100°C.
N-butyl aldehyde and iso-butyl aldehyde were separated by means of the distillation device of Comparative Example 1. With regard to this, used energy amount, recovery amount, reduction amount, reduction rate, and the purity of an n-butyl aldehyde/iso-butyl aldehyde product are summarized in Table 2 below.

**Comparative Example 2**

N-butyl aldehyde and iso-butyl aldehyde were separated in the same manner as in Example 1, except that the operation conditions of the first and second distillation columns were changed as disclosed in Table 2 below.
N-butyl aldehyde and iso-butyl aldehyde were separated by means of a distillation device of Comparative Example 2. With regard to this, used energy amount, recovery amount, reduction amount, reduction rate, and the purity of an n-butyl aldehyde/iso-butyl aldehyde product are summarized in Table 2 below.

**Comparative Example 3**

N-butyl aldehyde and iso-butyl aldehyde were separated in the same manner as in Example 1, except that the operation conditions of the first and second distillation columns were changed as disclosed in Table 2 below.
N-butyl aldehyde and iso-butyl aldehyde were separated by means of a distillation device of Comparative Example 3. With regard to this, used energy amount, recovery amount, reduction amount, reduction rate, and the purity of an n-butyl aldehyde/iso-butyl aldehyde product are summarized in Table 2 below.

**Comparative Example 4**

N-butyl aldehyde and iso-butyl aldehyde were separated in the same manner as in Example 1, except that the operation conditions of the first and second distillation columns were changed as disclosed in Table 2 below.
N-butyl aldehyde and iso-butyl aldehyde were separated by means of a distillation device of Comparative Example 4. With regard to this, used energy amount, recovery amount, reduction amount, reduction rate, and the purity of an n-butyl aldehyde/iso-butyl aldehyde product are summarized in Table 2 below.

**Comparative Example 5**

N-butyl aldehyde and iso-butyl aldehyde were separated in the same manner as in Example 1, except that the operation conditions of the first and second distillation columns were changed as disclosed in Table 3 below.
N-butyl aldehyde and iso-butyl aldehyde were separated by means of a distillation device of Comparative Example 5. With regard to this, used energy amount, recovery amount, reduction amount, reduction rate, and the purity of an n-butyl aldehyde/iso-butyl aldehyde product are summarized in Table 3 below.

**Comparative Example 6**

N-butyl aldehyde and iso-butyl aldehyde were separated in the same manner as in Example 1, except that the operation conditions of the first and second distillation columns were changed as disclosed in Table 3 below.

N-butyl aldehyde and iso-butyl aldehyde were separated by means of a distillation device of Comparative Example 6. With regard to this, used energy amount, recovery amount, reduction amount, reduction rate, and the purity of an n-butyl aldehyde/iso-butyl aldehyde product are summarized in Table 3 below.

## Comparative Example 7

N-butyl aldehyde and iso-butyl aldehyde were separated in the same manner as in Example 1, except that the operation conditions of the first and second distillation columns were changed as disclosed in Table 3 below.

N-butyl aldehyde and iso-butyl aldehyde were separated by means of a distillation device of Comparative Example 7. With regard to this, used energy amount, recovery amount, reduction amount, reduction rate, and the purity of an n-butyl aldehyde/iso-butyl aldehyde product are summarized in Table 3 below.

## Comparative Example 8

N-butyl aldehyde and iso-butyl aldehyde were separated in the same manner as in Example 1, except that the operation conditions of the first and second distillation columns were changed as disclosed in Table 3 below.

N-butyl aldehyde and iso-butyl aldehyde were separated by means of a distillation device of Comparative Example 8. With regard to this, used energy amount, recovery amount, reduction amount, reduction rate, and the purity of an n-butyl aldehyde/iso-butyl aldehyde product are summarized in Table 3 below.

[Table 1]

| | | Example 1 | Example 2 | Example 3 | Example 4 | Example 5 |
|---|---|---|---|---|---|---|
| Discharge rate of raw material (%) | First distillation column | 40 | 50 | 60 | 50 | 50 |
| | Second distillation column | 60 | 50 | 40 | 50 | 50 |
| Pressure of upper section ($kg/cm^2g$) | First distillation column | 0.02 | 0.02 | 0.02 | 0.07 | 0.1 |
| | Second distillation column | 2.5 | 2.5 | 2.5 | 2.5 | 2.5 |
| column temperature | First distillation column | 65/95 | 65/95 | 65/95 | 66/96 | 67/97 |
| (°C) (upper section/lower section) | Second distillation column | 105/129 | 105/129 | 105/129 | 105/129 | 105/129 |
| Energy (Gcal/hr) | First distillation column | 4.69 | 5.49 | 6.59 | 5.65 | 5.87 |
| | Second distillation column | 7.99 | 7.7 | 7.15 | 7.7 | 7.7 |
| | Recovery amount | 4.69 | 5.49 | 4.95 | 5.04 | 5.04 |
| | Total | 7.99 | 7.7 | 8.79 | 8.31 | 8.53 |
| | Reduction amount | 4.04 | 4.33 | 3.24 | 3.72 | 3.5 |
| | Energy reduction rate (%) | 33.6 | 36.0 | 26.9 | 30.9 | 29.1 |
| Product purity (n-BAL/iso-BAL) | | 99.7/99.0 | 99.7/99.0 | 99.7/99.0 | 99.7/99.0 | 99.7/99.0 |

[Table 2]

| | | Comparative Example 1 | Comparative Example 2 | Comparative Example 3 | Comparative Example 4 |
|---|---|---|---|---|---|
| Discharge rate of raw material (%) | First distillation column | - | 50 | 60 | 40 |
| | Second distillation column | 100 | 50 | 40 | 60 |
| Pressure of upper section ($Kg/cm^2g$) | First distillation column | - | 0.13 | 0.13 | 0.13 |
| | Second distillation column | 0.32 | 2.2 | 2.2 | 2.2 |
| Column temperature (°C) (upper section/ lower section) | First distillation column | - | 68/97 | 68/97 | 68/97 |
| | Second distillation column | 73/100 | 104/127 | 104/127 | 104/127 |
| Energy (Geal/hr) | First distillation column | - | 6.37 | 5.84 | 5.46 |
| | Second distillation column | 12.03 | 7.41 | 8.38 | 8.76 |
| | Recovery amount | - | 4.91 | 4.88 | 5.06 |
| | Total | 12.03 | 8.87 | 9.34 | 9.16 |
| | Reduction amount | - | 3.16 | 2.69 | 2.87 |
| | Energy reduction rate (%) | - | 26.3 | 22.4 | 23.9 |
| Product purity (n-BAL/iso-BAL) | | 99.7/99.0 | 99.7/99.0 | 99.7/99.0 | 99.7/99.0 |

[Table 3]

| | | Comparative Example 5 | Comparative Example 6 | Comparative Example 7 | Comparative Example 8 |
|---|---|---|---|---|---|
| Discharge rate of raw material (%) | First distillation column | 20 | 80 | 50 | 50 |
| | Second distillation column | 80 | 20 | 50 | 50 |

(continued)

| | | Comparative Example 5 | Comparative Example 6 | Comparative Example 7 | Comparative Example 8 |
|---|---|---|---|---|---|
| Pressure of upper section ($kg/cm^2g$) | First distillation column | 0.02 | 0.02 | 0.17 | 0.1 |
| | Second distillation column | 2.5 | 2.5 | 2.5 | 2.2 |
| Column temperature (°C) (upper section/ lower section) | First distillation column | 65/95 | 65/95 | 69/97 | 67/96 |
| | Second distillation column | 105/129 | 105/129 | 105/129 | 102/125 |
| Energy (Gcal/hr) | First distillation column | 2.20 | 8.79 | 7.07 | 7.02 |
| | Second distillation column | 14.31 | 3.58 | 7.70 | 7.65 |
| | Recovery amount | 2.20 | 2.21 | 4.72 | 4.99 |
| | Total | 14.20 | 10.16 | 10.05 | 9.31 |
| | Reduction amount | -2.17 | 1.87 | 1.98 | 2.35 |
| | Energy reduction rate(%) | - | 15.5 | 16.5 | 19.5 |
| Product purity (n-BAL/iso-BAL) | | 99.7/99.0 | 99.7/99.0 | 99.7/99.0 | 99.7/99.0 |

As shown in Tables 1 to 3, it can be confirmed that, when the isomer of n-butyl aldehyde is separated according to each of Examples 1 to 5, a total energy consumption amount is greatly decreased, compared to the comparative examples. Accordingly, it can be confirmed that, when the raw material was separated by means of the distillation device of each of Examples 1 to 5 of the present application, an energy reduction effect of up to 36.0% is achieved, compared to the case in which the distillation device of Comparative Example 1 is used.

In addition, it can be confirmed that, as shown in the examples and the comparative examples, n-butyl aldehyde and iso-butyl aldehyde may be separated in high purity and efficiency by controlling a temperature difference between the lower section of the first distillation column and the upper section of the second distillation column and the pressures of the upper sections of the first and second distillation columns within a specific range.

In addition, as shown in Comparative Examples 5 and 6, it can be confirmed that n-butyl aldehyde and iso-butyl aldehyde may be separated in high purity and efficiency by controlling a ratio of the discharge rate into each of the first and second distillation columns within a specific range.

## Example 6

N-butyl aldehyde and iso-butyl aldehyde were separated by means of a distillation device illustrated in FIG. 2. In particular, a raw material including n-butyl aldehyde and iso-butyl aldehyde was introduced into a first distillation column with a theoretical plate number of 100.

A portion of a first column top stream discharged from an upper section of the first distillation column was refluxed in the upper section of the first distillation column via a first condenser. A portion of the remainder of the first

column top stream was separated as a product including iso-butyl aldehyde and stored. A portion of a first column bottom stream discharged from a lower section of the first distillation column was refluxed in the lower section of the first distillation column via a first reboiler. A second column bottom stream discharged from the lower section of the first distillation column was introduced into a second distillation column. A third column bottom stream discharged from the lower section of the first distillation column was introduced into a heat exchanger and heat-exchanged with a second column top stream of the second distillation column introduced into the heat exchanger, followed by being refluxed in the lower section of the first distillation column via the heat exchanger. In this case, operation pressure of the upper section of the first distillation column was adjusted to 0.07 $Kg/cm^2g$ and operation temperature thereof was adjusted to 65 °C. Operation temperature of the lower section of the first distillation column was adjusted to 96°C.

Meanwhile, the second column top stream discharged from an upper section of the second distillation column was introduced into the heat exchanger and heat-exchanged with the third column bottom stream. Subsequently, a portion of the second column top stream having passed through the heat exchanger and a second condenser was refluxed in the upper section of the second distillation column, and a portion of the remainder of the second column top stream was separated as a product including n-butyl aldehyde. In this case, the purity of n-butyl aldehyde was 99.9%. A fourth column bottom stream discharged from a lower section of the second distillation column was refluxed in the lower section of the second distillation column via a second reboiler, and a fifth column bottom stream discharged from the lower section of the second distillation column was separated as a product including n-butyl aldehyde. In this case, operation pressure of the upper section of the second distillation column was adjusted to 1.4 $Kg/cm^2g$, and operation temperature thereof was adjusted to 105°C. Operation temperature of the lower section of the second distillation column was adjusted to 120°C.

N-butyl aldehyde and iso-butyl aldehyde were separated by means of the distillation device of Example 6. With regard to this, used energy amount, recovery amount, reduction amount, reduction rate, and the purity of an n-butyl aldehyde/iso-butyl aldehyde product are summarized in Table 4 below..

## Example 7

N-butyl aldehyde and iso-butyl aldehyde were separated in the same manner as in Example 6, except that the operation conditions of the first and second distillation columns were changed as disclosed in Table 3 below.

N-butyl aldehyde and iso-butyl aldehyde were separated by means of a distillation device of Example 7. With regard to this, used energy amount, recovery amount, reduction amount, reduction rate, and the purity of an n-butyl aldehyde/iso-butyl aldehyde product are summarized in Table 4 below.

## Example 8

N-butyl aldehyde and iso-butyl aldehyde were separated in the same manner as in Example 6, except that the operation conditions of the first and second distillation columns were changed as disclosed in Table 3 below.

N-butyl aldehyde and iso-butyl aldehyde were separated by means of a distillation device of Example 8. With regard to this, used energy amount, recovery amount, reduction amount, reduction rate, and the purity of an n-butyl aldehyde/iso-butyl aldehyde product are summarized in Table 4 below.

## Comparative Example 9

N-butyl aldehyde and iso-butyl aldehyde were separated in the same manner as in Example 6, except that the operation conditions of the first and second distillation columns were changed as disclosed in Table 5 below.

N-butyl aldehyde and iso-butyl aldehyde were separated by means of a distillation device of Comparative Example 9. With regard to this, used energy amount, recovery amount, reduction amount, reduction rate, and the purity of an n-butyl aldehyde/iso-butyl aldehyde product are summarized in Table 5 below.

## Comparative Example 10

N-butyl aldehyde and iso-butyl aldehyde were separated in the same manner as in Example 6, except that the operation conditions of the first and second distillation columns were changed as disclosed in Table 5 below.

N-butyl aldehyde and iso-butyl aldehyde were separated by means of a distillation device of Comparative Example 10. With regard to this, used energy amount, recovery amount, reduction amount, reduction rate, and the purity of an n-butyl aldehyde/iso-butyl aldehyde product are summarized in Table 5 below.

## Comparative Example 11

N-butyl aldehyde and iso-butyl aldehyde were separated in the same manner as in Example 6, except that the operation conditions of the first and second distillation columns were changed as disclosed in Table 5 below.
N-butyl aldehyde and iso-butyl aldehyde were separated by means of a distillation device of Comparative Example 11. With regard to this, used energy amount, recovery amount, reduction amount, reduction rate, and the purity of an n-butyl aldehyde/iso-butyl aldehyde product are summarized in Table 5 below.

## Comparative Example 12

N-butyl aldehyde and iso-butyl aldehyde were separated in the same manner as in Example 6, except that the operation conditions of the first and second distillation columns were changed as disclosed in Table 6 below.
N-butyl aldehyde and iso-butyl aldehyde were separated by means of a distillation device of Comparative Example 12. With regard to this, used energy amount, recovery amount, reduction amount, reduction rate, and the purity of an n-butyl aldehyde/iso-butyl aldehyde product are summarized in Table 6 below.

## Comparative Example 13

N-butyl aldehyde and iso-butyl aldehyde were separated in the same manner as in Example 6, except that the operation conditions of the first and second distillation columns were changed as disclosed in Table 6 below.
N-butyl aldehyde and iso-butyl aldehyde were separated by means of a distillation device of Comparative Example 13. With regard to this, used energy amount, recovery amount, reduction amount, reduction rate, and the purity of an n-butyl aldehyde/iso-butyl aldehyde product are summarized in Table 6 below.

[Table 4]

| | | Example 6 | Example 7 | Example 8 |
|---|---|---|---|---|
| Pressure of upper section (kg/cm$^2$g) | First distillation column | 0.07 | 0.07 | 0.1 |
| | Second distillation column | 1.4 | 1.8 | 2.1 |
| Column temperature (°C) (upper section/lower section) | First distillation column | 66/96 | 66/96 | 67/97 |
| | Second distillation column | 105/120 | 110/124 | 114/128 |
| Energy (Gcal/hr) | First distillation column | 10.91 | 10.91 | 11.45 |
| | Second distillation column | 5.71 | 5.82 | 6.01 |
| | Recovery amount | 4.59 | 4.72 | 4.80 |
| | Total | 12.03 | 12.01 | 12.66 |
| | Reduction amount | 2.34 | 2.36 | 1.71 |
| | Energy reduction rate (%) | 16.3 | 16.4 | 11.9 |
| Product purity (n-BAL/iso-BAL) | | 99.9/99.3 | 99.9/99.3 | 99.9/99.3 |

[Table 5]

| | | Comparative Example 9 | Comparative Example 10 | Comparative Example 11 |
|---|---|---|---|---|
| Pressure of upper section ($kg/cm^2g$) | First distillation column | 0.34 | 0.18 | 0.052 |
| | Second distillation column | 0.15 | 1.4 | 1.2 |
| Column temperature (°C) (upper section/lower | First distillation column | 73/100 | 69/98 | 66/95 |
| | Second | 79/97 | 105/120 | 101/116 |
| section) | distillation column | | | |
| Energy (Gcal/hr) | First distillation column | 12.07 | 11.79 | 10.91 |
| | Second distillation column | 2.3 | 5.67 | 5.65 |
| | Recovery amount | - | 3.94 | 3.43 |
| | Total | 14.37 | 13.52 | 13.13 |
| | Reduction amount | | 0.85 | 1.24 |
| | Energy reduction rate (%) | - | 5.9 | 8.6 |
| Product purity (n-BAL/iso-BAL) | | 99.9/99.3 | 99.9/99.3 | 99.9/99.3 |

[Table 6]

| | i | Comparative Example 12 | Comparative Example 13 |
|---|---|---|---|
| Pressure of upper section ($kg/cm^2g$) | First distillation column | 0.11 | 0.3 |
| | Second distillation column | 1.35 | 1.6 |
| Column temperature (°C) (upper section/ lower section) | First distillation column | 67/96 | 72/100 |
| | Second distillation column | 104/167 | 108/170 |
| Energy (Gcal/hr) | First distillation column | 11.5 | 11.9 |
| | Second distillation column | 5.60 | 6.32 |
| | Recovery amount | 4.38 | 3.88 |
| | Total | 12.72 | 14.34 |
| | Reduction amount | 1.65 | 0.03 |
| | Energy reduction rate (%) | 11.5 | 0.2 |
| Product purity (n-BAL/iso-BAL) | | 99.9/99.3 | 99.9/99.3 |

As shown in Tables 4 to 6, it can be confirmed that, when the isomer of n-butyl aldehyde is separated according to each of Examples 6 to 8, a total energy consumption amount is greatly decreased, compared to the comparative examples. Accordingly, it can be confirmed that, when the raw material was separated by means of the distillation device of each of Examples 6 to 8 of the present application, an energy reduction effect of up to 16.4% is achieved, compared to the case in which the distillation device of Comparative Example 5 is used.

In addition, it can be confirmed that, as shown in the examples and the comparative examples, n-butyl aldehyde may be separated in high purity and efficiency by controlling a temperature difference between the lower section of the first distillation column and the upper section of the second distillation column and the pressures of the upper sections of the first and second distillation columns, within a specific range.

## Claims

1. A method of using a distillation device, comprising a first distillation unit that comprises a first condenser, a first reboiler and a first distillation column; a second distillation unit that comprises a second condenser, a second reboiler, and a second distillation column; and a heat exchanger,
wherein a raw material comprising a compound represented by Formula 1 below and an isomer of the compound is introduced into a first supply port of the first distillation column and/or a second supply port of the second distillation column,
the raw material introduced into the first supply port of the first distillation column is separately discharged into each of a first column top stream discharged from an upper section of the first distillation column; and first, second and third column bottom streams separately discharged from a lower section of the first distillation column,
the first column top stream is introduced into the first condenser, and a portion or all of the first column top stream via the first condenser is refluxed in the upper section of the first distillation column,
the first column bottom stream is introduced into the first reboiler, and the first column bottom stream via the first reboiler is refluxed in the lower section of the first distillation column,
the stream introduced into the second supply port of the second distillation column is separately discharged to each of a second column top stream discharged from an upper section of the second distillation column; and fourth and fifth column bottom streams discharged from a lower section of the second distillation column,
the fourth column bottom stream is introduced into the second reboiler, and the fourth column bottom stream via the second reboiler is refluxed in the lower section of the second distillation column,
the third column bottom stream and the second column top stream are introduced into the heat exchanger and exchange heat therebetween, the third column bottom stream via the heat exchanger is refluxed in the lower section of the first distillation column, the second column top stream via the heat exchanger is introduced into the second condenser, and a portion or all of the second column top stream via the second condenser is refluxed in the upper section of the second distillation column, and
Equations 1 and 2 are satisfied,
wherein the compound represented by Formula 1 is n-butyl aldehyde, and the isomer of the compound is iso-butyl aldehyde:

[Formula 1]

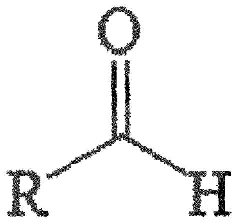

wherein R is a $C_1$ to $C_{12}$ alkyl group;

[Equation 1]

$$T_{t-2} - T_{b-3} \geq 8 ℃$$

[Equation 2]
$$P_2/P_1 \geq 20$$

wherein $T_{t-2}$ indicates a temperature of the second column top stream, and $T_{b-3}$ indicates a temperature of the third column bottom stream, and
$P_1$ indicates a pressure of the upper section of the first distillation column ($kg/cm^2g$), and $P_2$ indicates a pressure of the upper section of the second distillation column ($kg/cm^2g$).

2. The method according to claim 1, wherein the raw material is introduced into each of the first supply port of the first distillation column and the second supply port of the second distillation column.

3. The method according to claim 2, wherein a content of the iso-butyl aldehyde in each of the first column top stream and the second column top stream is 90% or more, and a content of the n-butyl aldehyde in each of the second column bottom stream and the fifth column bottom stream is 90% or more.

4. The method according to claim 2, wherein the distillation device satisfies Equation 3 below:

[Equation 3]
$$0.3 \leq F_1/F_2 \leq 3.0,$$

wherein $F_1$ indicates a discharge rate of the raw material (ton/hr) introduced into the first supply port of the first distillation column, and $F_2$ indicates a discharge rate of the raw material (ton/hr) introduced into the second supply port of the second distillation column.

5. The method according to claim 1, wherein a pressure of the upper section of the first distillation column is 0.01 to 0.1 $kg/cm^2g$ and a pressure of the upper section of the second distillation column is 2.3 to 2.7 $kg/cm^2g$.

6. The method according to claim 1, wherein a temperature of the upper section of the first distillation column is 60 to 70°C and a temperature of the lower section of the second distillation column is 120 to 140°C.

7. The method according to claim 1, wherein a temperature of the lower section of the first distillation column is 90 to 100°C and a temperature of the upper section of the second distillation column is 100 to 110°C.

8. The method according to claim 1, wherein the raw material is supplied to the first supply port of the first distillation column, and the second column bottom stream of the first distillation column is supplied to the second supply port of the second distillation column.

9. The method according to claim 8, wherein a content of the iso-butyl aldehyde in the first column top stream is 90% or more, and a content of the n-butyl aldehyde in the second column top stream is 90% or more.

10. The method according to claim 8, wherein a portion of the fifth column bottom stream discharged from the lower section of the second distillation column is introduced into the lower section of the first distillation column.

11. The method according to claim 8, wherein a pressure of the upper section of the first distillation column is 0.01 to 0.1 $kg/cm^2g$ and a pressure of the upper section of the second distillation column is 1.0 to 2.0 $kg/cm^2g$.

12. The method according to claim 8, wherein a temperature of the upper section of the first distillation column is 60 to 70°C and a temperature of the lower section of the second distillation column is 120 to 140°C.

13. The method according to claim 8, wherein a temperature of the lower section of the first distillation column is 90 to 100°C and a temperature of the upper section of the second distillation column is 100 to 110°C.

14. A method of preparing a compound represented by Formula 1, the method comprising: introducing a raw material comprising the compound represented by Formula 1 below and an isomer of the compound into each of a first supply port of a first distillation column and a second supply port of a second distillation column;
discharging the raw material introduced into the first supply port to each of a first column top stream discharged

from an upper section of the first distillation column; and first, second and third column bottom streams discharged from a lower section of the first distillation column;
discharging the raw material introduced into the second supply port to each of a second column top stream discharged from an upper section of the second distillation column; and fourth and fifth column bottom streams discharged from a lower section of the second distillation column;
heat-exchanging the second column top stream with the third column bottom stream; and
separating the compound represented by Formula 1 from the lower section of the first distillation column, and the isomer of the compound represented by Formula 1 from the upper section of the first distillation column and the upper section of the second distillation column,
wherein Equations 1 and 2 below are satisfied,
wherein the compound represented by Formula 1 is n-butyl aldehyde, and the isomer of the compound is iso-butyl aldehyde:

[Formula 1]

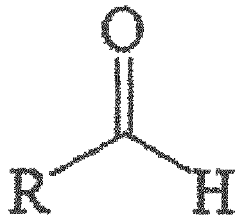

wherein R is a $C_1$ to $C_{12}$ alkyl group;

[Equation 1]

$$T_{t-2} - T_{b-3} \geq 8\,^{\circ}C$$

[Equation 2]

$$P_2/P_1 \geq 20$$

wherein $T_{t-2}$ indicates a temperature of the second column top stream, and $T_{b-3}$ indicates a temperature of the third column bottom stream, and
$P_1$ indicates a pressure of the upper section of the first distillation column (kg/cm$^2$g), and $P_2$ indicates a pressure of the upper section of the second distillation column (kg/cm$^2$g).

**15.** A method of preparing a compound represented by Formula 1, the method comprising: introducing a raw material comprising the compound represented by Formula 1 below and an isomer of the compound into a first supply port of a first distillation column;
discharging the introduced raw material to each of a first column top stream discharged from an upper section of the first distillation column; and first, second and third column bottom streams discharged from a lower section of the first distillation column;
introducing the first column bottom stream into a second supply port of a second distillation column,
discharging the stream introduced into the second supply port to each of a second column top stream discharged from an upper section of the second distillation column; and fourth and fifth column bottom streams discharged from a lower section of the second distillation column;
heat-exchanging the second column top stream with the third column bottom stream; and
separating the compound represented by Formula 1 from the upper section of the second distillation column, and the isomer of the compound represented by Formula 1 from the upper section of the first distillation column,
wherein Equations 1 and 2 below are satisfied,
wherein the compound represented by Formula 1 is n-butyl aldehyde, and the isomer of the compound is iso-butyl aldehyde:

[Formula 1]

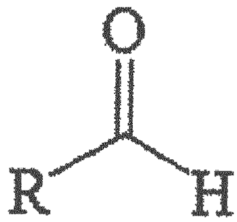

wherein R is a $C_1$ to $C_{12}$ alkyl group;

[Equation 1]

$$T_{t-2} - T_{b-3} \geq 8 ℃$$

[Equation 2]

$$P_2/P_1 \geq 20$$

wherein $T_{t-2}$ indicates a temperature of the second column top stream, and $T_{b-3}$ indicates a temperature of the third column bottom stream, and

$P_1$ indicates a pressure of the upper section of the first distillation column ($kg/cm^2g$), and $P_2$ indicates a pressure of the upper section of the second distillation column ($kg/cm^2g$).

## Patentansprüche

1. Verfahren der Verwendung einer Destillationsvorrichtung, umfassend eine erste Destillationseinheit, die einen ersten Kondensator, einen ersten Verdampfer und eine erste Destillationskolonne umfasst; eine zweite Destillationseinheit, die einen zweiten Kondensator, einen zweiten Verdampfer und eine zweite Destillationskolonne umfasst; und einen Wärmetauscher,
   wobei ein Rohmaterial, das eine Verbindung der nachstehenden Formel 1 und ein Isomer der Verbindung umfasst, in eine erste Zuführöffnung der ersten Destillationskolonne und/oder eine zweite Zuführöffnung der zweiten Destillationskolonne eingeführt wird,
   das in die erste Zuführöffnung der ersten Destillationskolonne eingebrachte Rohmaterial separat in jeden eines ersten Kolonnenoberstroms, der aus einem oberen Abschnitt der ersten Destillationskolonne abgeleitet wird; und eines ersten, zweiten und dritten Kolonnenunterstroms, die separat aus einem unteren Abschnitt der ersten Destillationskolonne abgeleitet werden, eingeleitet wird,
   der erste Kolonnenoberstrom in den ersten Kondensator eingeleitet wird, und ein Teil des oder der gesamte erste Kolonnenoberstrom über den ersten Kondensator in den oberen Abschnitt der ersten Destillationskolonne refluxiert wird,
   der erste Kolonnenbodenstrom in den ersten Verdampfer eingeleitet wird, und der erste Kolonnenbodenstrom über den ersten Verdampfer im unteren Abschnitt der ersten Destillationskolonne refluxiert wird,
   der in die zweite Zuführöffnung der zweiten Destillationskolonne eingeleitete Strom separat an jeden eines zweiten Kolonnenoberstroms, der aus einem oberen Abschnitt der zweiten Destillationskolonne abgeführt wird; und eines vierten und fünften Kolonnenunterstroms, die aus einem unteren Abschnitt der zweiten Destillationskolonne abgeführt werden, der vierte Kolonnenbodenstrom in den zweiten Verdampfer eingeleitet wird, und der vierte Kolonnenbodenstrom über den zweiten Verdampfer im unteren Abschnitt der zweiten Destillationskolonne zurückgeführt wird, abgeleitet wird,
   der dritte Kolonnenunterstrom und der zweite Kolonnenoberstrom in den Wärmetauscher eingeleitet werden und dazwischen Wärme austauschen, der dritte Kolonnenunterstrom über den Wärmetauscher im unteren Abschnitt der ersten Destillationskolonne refluxiert wird, der zweite Kolonnenoberstrom über den Wärmetauscher in den zweiten Kondensator eingeleitet wird und ein Teil oder der gesamte zweite Kolonnenoberstrom über den zweiten Kondensator im oberen Abschnitt der zweiten Destillationskolonne refluxiert wird, und
   die Gleichungen 1 und 2 erfüllt sind,
   wobei die durch Formel 1 dargestellte Verbindung n-Butylaldehyd ist und das Isomer der Verbindung Isobutylaldehyd ist:

[Formel 1]

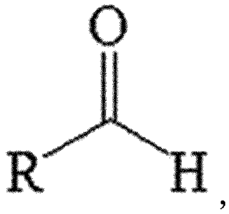

wobei R eine $C_1$-$C_{12}$-Alkylgruppe ist;

[Gleichung 1]

$$T_{t-2} - T_{b-3} \geq 8°C$$

[Gleichung 2]

$$P_2/P_1 \geq 20,$$

wobei $T_{t-2}$ eine Temperatur des zweiten oberen Säulenstroms anzeigt und $T_{b-3}$ eine Temperatur des dritten unteren Säulenstroms anzeigt, und
$P_1$ einen Druck des oberen Teils der ersten Destillationskolonne (kg/cm$^2$g) und P2 einen Druck des oberen Teils der zweiten Destillationskolonne (kg/cm$^2$g) anzeigt.

2. Verfahren nach Anspruch 1, wobei das Rohmaterial in jede der ersten Zuführöffnung der ersten Destillationskolonne und der zweiten Zuführöffnung der zweiten Destillationskolonne eingebracht wird.

3. Verfahren nach Anspruch 2, wobei ein Gehalt des Isobutylaldehyds in jedem des ersten Kolonnenoberstroms und des zweiten Kolonnenoberstroms 90% oder mehr beträgt und ein Gehalt des n-Butylaldehyds in jedem des zweiten Kolonnenunterstroms und des fünften Kolonnenunterstroms 90% oder mehr beträgt.

4. Verfahren nach Anspruch 2, wobei die Destillationsvorrichtung die folgende Gleichung 3 erfüllt:

[Gleichung 3]

$$0{,}3 \leq F_1/F_2 \leq 3{,}0,$$

wobei $F_1$ eine Austragsrate des Rohmaterials (ton/h) anzeigt, das in die erste Zuführöffnung der ersten Destillationskolonne eingebracht ist, und $F_2$ eine Austragsrate des in die zweite Zuführöffnung der zweiten Destillationskolonne eingebrachten Rohmaterials (ton/h) anzeigt.

5. Verfahren nach Anspruch 1, wobei ein Druck des oberen Teils der ersten Destillationskolonne 0,01 bis 0,1 kg/cm$^2$g und ein Druck des oberen Teils der zweiten Destillationskolonne 2,3 bis 2,7 kg/cm$^2$g beträgt.

6. Verfahren nach Anspruch 1, wobei eine Temperatur des oberen Teils der ersten Destillationskolonne 60 bis 70°C und eine Temperatur des unteren Teils der zweiten Destillationskolonne 120 bis 140°C beträgt.

7. Verfahren nach Anspruch 1, wobei eine Temperatur des unteren Teils der ersten Destillationskolonne 90 bis 100°C und eine Temperatur des oberen Teils der zweiten Destillationskolonne 100 bis 110°C beträgt.

8. Verfahren nach Anspruch 1, wobei das Rohmaterial der ersten Zuführöffnung der ersten Destillationskolonne zugeführt wird und der zweite Kolonnenbodenstrom der ersten Destillationskolonne der zweiten Zuführöffnung der zweiten Destillationskolonne zugeführt wird.

**9.** Verfahren nach Anspruch 8, wobei ein Gehalt des Isobutylaldehyds im ersten Kolonnenoberstrom 90% oder mehr und ein Gehalt des n-Butylaldehyds im zweiten Kolonnenoberstrom 90% oder mehr beträgt.

**10.** Verfahren nach Anspruch 8, wobei ein Teil des fünften Kolonnenbodenstroms, der aus dem unteren Abschnitt der zweiten Destillationskolonne abgeleitet wird, in den unteren Abschnitt der ersten Destillationskolonne eingebracht wird.

**11.** Verfahren nach Anspruch 8, wobei ein Druck des oberen Teils der ersten Destillationskolonne 0,01 bis 0,1 kg/cm$^2$g und ein Druck des oberen Teils der zweiten Destillationskolonne 1,0 bis 2,0 kg/cm$^2$g beträgt.

**12.** Verfahren nach Anspruch 8, wobei eine Temperatur des oberen Teils der ersten Destillationskolonne 60 bis 70°C und eine Temperatur des unteren Teils der zweiten Destillationskolonne 120 bis 140°C beträgt.

**13.** Verfahren nach Anspruch 8, wobei eine Temperatur des unteren Teils der ersten Destillationskolonne 90 bis 100°C und eine Temperatur des oberen Teils der zweiten Destillationskolonne 100 bis 110°C beträgt.

**14.** Verfahren zum Herstellen einer Verbindung, dargestellt durch die Formel 1, das Verfahren umfassend: Einführen eines Rohmaterials, das die Verbindung der folgenden Formel 1 und ein Isomer der Verbindung umfasst, in jede einer ersten Zuführöffnung einer ersten Destillationskolonne und einer zweiten Zuführöffnung einer zweiten Destillationskolonne;
Entladen des in die erste Zuführöffnung eingeleiteten Rohmaterials in jeden des ersten Kolonnenoberstroms, der aus einem oberen Abschnitt der ersten Destillationskolonne ausgeleitet wird; und des ersten, zweiten und dritten Kolonnenunterstroms, die aus einem unteren Abschnitt der ersten Destillationskolonne ausgeleitet werden;
Entladen des in die zweite Zuführöffnung eingeleiteten Rohmaterials in jeden der oberen Ströme einer zweiten Kolonne, die aus einem oberen Abschnitt der zweiten Destillationskolonne ausgeleitet werden; und der unteren Ströme der vierten und fünften Kolonne, die aus einem unteren Abschnitt der zweiten Destillationskolonne ausgeleitet werden;
Wärmeaustausch des zweiten oberen Säulenstroms mit dem dritten unteren Säulenstrom; und
Trennen der Verbindung der Formel 1 vom unteren Abschnitt der ersten Destillationskolonne und des Isomers der Verbindung der Formel 1 vom unteren Abschnitt der ersten Destillationskolonne und dem oberen Abschnitt der zweiten Destillationskolonne,
wobei die folgenden Gleichungen 1 und 2 unten erfüllt sind,
wobei die durch Formel 1 dargestellte Verbindung n-Butylaldehyd ist und das Isomer der Verbindung Isobutylaldehyd ist;

[Formel 1]

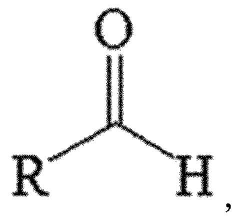

wobei R eine $C_1$-$C_{12}$-Alkylgruppe ist;

[Gleichung 1]

$$T_{t-2} - T_{b-3} \geq 8°C$$

[Gleichung 2]

$$P_2/P_1 \geq 20,$$

wobei $T_{t-2}$ eine Temperatur des zweiten oberen Säulenstroms anzeigt und $T_{b-3}$ eine Temperatur des dritten unteren Säulenstroms anzeigt, und
$P_1$ einen Druck des oberen Teils der ersten Destillationskolonne ($kg/cm^2g$) und P2 einen Druck des oberen Teils der zweiten Destillationskolonne ($kg/cm^2g$) anzeigt.

**15.** Verfahren zum Herstellen einer Verbindung der Formel 1, wobei das Verfahren umfasst: Einführen eines Rohmaterials, das die Verbindung der folgenden Formel 1 und ein Isomer der Verbindung umfasst, in eine erste Zuführöffnung einer ersten Destillationskolonne;
Entladen des eingeleiteten Rohmaterials in jeden des ersten Kolonnenoberstroms, der aus einem oberen Abschnitt der ersten Destillationskolonne ausgeleitet wird; und des ersten, zweiten und dritten Kolonnenunterstroms, die aus einem unteren Abschnitt der ersten Destillationskolonne ausgeleitet werden;
Einführen des ersten Kolonnenbodenstroms in eine zweite Zuführöffnung einer zweiten Destillationskolonne,
Entladen des in die zweite Zuführöffnung eingeleiteten Stroms in jeden der oberen Ströme einer zweiten Kolonne, die aus einem oberen Abschnitt der zweiten Destillationskolonne ausgeleitet werden; und der unteren Ströme der vierten und fünften Kolonne, die aus einem unteren Abschnitt der zweiten Destillationskolonne ausgeleitet werden;
Wärmeaustausch des zweiten oberen Säulenstroms mit dem dritten unteren Säulenstrom; und
Trennen der Verbindung der Formel 1 vom oberen Abschnitt der zweiten Destillationskolonne und des Isomers der Verbindung der Formel 1 vom oberen Abschnitt der ersten Destillationskolonne,
wobei die folgenden Gleichungen 1 und 2 unten erfüllt sind,
wobei die durch Formel 1 dargestellte Verbindung n-Butylaldehyd ist und das Isomer der Verbindung Isobutylaldehyd ist:

[Formel 1]

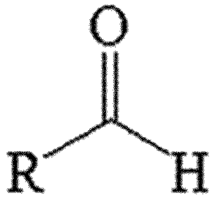,

wobei R eine $C_1$-$C_{12}$-Alkylgruppe ist;

[Gleichung 1]

$$T_{t-2} - T_{b-3} \geq 8°C$$

[Gleichung 2]

$$P_2/P_1 \geq 20,$$

wobei $T_{t-2}$ eine Temperatur des zweiten oberen Säulenstroms anzeigt und $T_{b-3}$ eine Temperatur des dritten unteren Säulenstroms anzeigt, und
$P_1$ einen Druck des oberen Teils der ersten Destillationskolonne ($kg/cm^2g$) und $P_2$ einen Druck des oberen Teils der zweiten Destillationskolonne ($kg/cm^2g$) anzeigt.

**Revendications**

**1.** Procédé d'utilisation d'un dispositif de distillation, comprenant :

une première unité de distillation qui comprend un premier condenseur, un premier rebouilleur et une première colonne de distillation ;
une deuxième unité de distillation qui comprend un deuxième condenseur, un deuxième rebouilleur et une

deuxième colonne de distillation ; et
un échangeur de chaleur,
dans lequel une matière première comprenant un composé représenté par la Formule 1 ci-dessous et un isomère du composé est introduite dans un premier orifice d'alimentation de la première colonne de distillation et/ou un deuxième orifice d'alimentation de la deuxième colonne de distillation,
la matière première introduite dans le premier orifice d'alimentation de la première colonne de distillation est déchargée séparément dans chacun des flux supérieurs de la première colonne déchargés d'une section supérieure de la première colonne de distillation ; et des flux inférieurs des première, deuxième et troisième colonnes déchargés séparément d'une section inférieure de la première colonne de distillation,
le flux supérieur de la première colonne est introduit dans le premier condenseur, et une partie ou la totalité du flux supérieur de la première colonne via le premier condenseur est refoulée dans la section supérieure de la première colonne de distillation,
le flux inférieur de la première colonne est introduit dans le premier rebouilleur, et le flux inférieur de la première colonne via le premier rebouilleur est refoulé dans la section inférieure de la première colonne de distillation,
le flux introduit dans le deuxième orifice d'alimentation de la deuxième colonne de distillation est déchargé séparément vers chacun des flux supérieurs de la deuxième colonne déchargés d'une section supérieure de la deuxième colonne de distillation ; et des flux inférieurs des quatrième et cinquième colonnes déchargés d'une section inférieure de la deuxième colonne de distillation,
le flux inférieur de la quatrième colonne est introduit dans le deuxième rebouilleur, et le flux inférieur de la quatrième colonne via le deuxième rebouilleur est refoulé dans la section inférieure de la deuxième colonne de distillation,
le flux inférieur de la troisième colonne et le flux supérieur de la deuxième colonne sont introduits dans l'échangeur de chaleur et échangent la chaleur entre eux, le flux inférieur de la troisième colonne via l'échangeur de chaleur est refoulé dans la section inférieure de la première colonne de distillation, le flux supérieur de la deuxième colonne via l'échangeur de chaleur est introduit dans le deuxième condenseur, et une partie ou la totalité du flux supérieur de la deuxième colonne via le deuxième condenseur est refoulée dans la section supérieure de la deuxième colonne de distillation, et
les Équations 1 et 2 sont satisfaites,
dans lequel le composé représenté par la Formule 1 est un aldéhyde de n-butyle, et l'isomère du composé est un aldéhyde d'isobutyle :

[Formule 1]

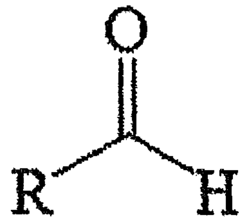

dans lequel R est un groupe alkyle en $C_1$ à $C_{12}$ ;

[Équation 1]

$$T_{t-2} - T_{b-3} \geq 8°C$$

[Équation 2]

$$P_2/P_1 \geq 20$$

dans lequel $T_{t-2}$ indique une température du flux supérieur de la deuxième colonne et $T_{b-3}$ indique une température du flux inférieur de la troisième colonne, et
$P_1$ indique une pression de la section supérieure de la première colonne de distillation (kg/cm$^2$g) et $P_2$ indique une pression de la section supérieure de la deuxième colonne de distillation (kg/cm$^2$g).

**2.** Procédé selon la revendication 1, dans lequel la matière première est introduite dans chaque premier orifice d'alimentation de la première colonne de distillation et chaque deuxième orifice d'alimentation de la deuxième colonne de distillation.

**3.** Procédé selon la revendication 2, dans lequel une teneur en aldéhyde d'isobutyle dans chaque flux supérieur de la première colonne et chaque flux supérieur de la deuxième colonne est égale ou supérieure à 90%, et une teneur en aldéhyde de n-butyle dans chaque flux inférieur de la deuxième colonne et chaque flux inférieur de la cinquième colonne est égale ou supérieure à 90%.

**4.** Procédé selon la revendication 2, dans lequel le dispositif de distillation satisfait à l'Équation 3 ci-dessous :

[Équation 3]

$$0,3<F_1/F_2 \leq 3,0,$$

dans lequel $F_1$ indique un débit de décharge de la matière première (tonne/h) introduite dans le premier orifice d'alimentation de la première colonne de distillation, et $F_2$ indique un débit de décharge de la matière première (tonne/h) introduite dans le deuxième orifice d'alimentation de la deuxième colonne de distillation.

**5.** Procédé selon la revendication 1, dans lequel une pression de la section supérieure de la première colonne de distillation est comprise entre 0,01 et 0,1 $kg/cm^2g$ et une pression de la section supérieure de la deuxième colonne de distillation est comprise entre 2,3 et 2,7 $kg/cm^2g$.

**6.** Procédé selon la revendication 1, dans lequel une température de la section supérieure de la première colonne de distillation est comprise entre 60 et 70°C et une température de la section inférieure de la deuxième colonne de distillation est comprise entre 120 et 140°C.

**7.** Procédé selon la revendication 1, dans lequel une température de la section inférieure de la première colonne de distillation est comprise entre 90 et 100°C et une température de la section supérieure de la deuxième colonne de distillation est comprise entre 100 et 110°C.

**8.** Procédé selon la revendication 1, dans lequel la matière première est fournie au premier orifice d'alimentation de la première colonne de distillation, et le flux inférieur de la deuxième colonne de la première colonne de distillation est fourni au deuxième orifice d'alimentation de la deuxième colonne de distillation.

**9.** Procédé selon la revendication 8, dans lequel une teneur en aldéhyde d'isobutyle dans le flux supérieur de la première colonne est égale ou supérieure à 90%, et une teneur en aldéhyde de n-butyle dans le flux supérieur de la deuxième colonne est égale ou supérieure à 90%.

**10.** Procédé selon la revendication 8, dans lequel une partie du flux inférieur de la cinquième colonne déchargé de la section inférieure de la deuxième colonne de distillation est introduit dans la section inférieure de la première colonne de distillation.

**11.** Procédé selon la revendication 8, dans lequel une pression de la section supérieure de la première colonne de distillation est comprise entre 0,01 et 0,1 $kg/cm^2g$ et une pression de la section supérieure de la deuxième colonne de distillation est comprise entre 1,0 et 2,0 $kg/cm^2g$.

**12.** Procédé selon la revendication 8, dans lequel une température de la section supérieure de la première colonne de distillation est comprise entre 60 et 70°C et une température de la section inférieure de la deuxième colonne de distillation est comprise entre 120 et 140°C.

**13.** Procédé selon la revendication 8, dans lequel une température de la section inférieure de la première colonne de distillation est comprise entre 90 et 100°C et une température de la section supérieure de la deuxième colonne de distillation est comprise entre 100 et 110°C.

**14.** Procédé de préparation d'un composé représenté par la Formule 1, le procédé consistant à :

introduire une matière première comprenant le composé représenté par la Formule 1 ci-dessous et un isomère du composé dans chaque premier orifice d'alimentation d'une première colonne de distillation et chaque deuxième orifice d'alimentation d'une deuxième colonne de distillation ;
décharger la matière première introduite dans le premier orifice d'alimentation vers chacun des flux supérieurs de la première colonne déchargés d'une section supérieure de la première colonne de distillation ; et des flux inférieurs des première, deuxième et troisième colonnes déchargés d'une section inférieure de la première colonne de distillation ;
décharger la matière première introduite dans le deuxième orifice d'alimentation vers chacun des flux supérieurs de la deuxième colonne déchargés d'une section supérieure de la deuxième colonne de distillation ; et des flux inférieurs des quatrième et cinquième colonnes déchargés d'une section inférieure de la deuxième colonne de distillation ;
échanger la chaleur du flux supérieur de la deuxième colonne avec le flux inférieur de la troisième colonne ; et séparer le composé représenté par la Formule 1 de la section inférieure de la première colonne de distillation, et l'isomère du composé représenté par la Formule 1 de la section supérieure de la première colonne de distillation et la section supérieure de la deuxième colonne de distillation,
dans lequel les Équations 1 et 2 ci-dessous sont satisfaites,
dans lequel le composé représenté par la Formule 1 est un aldéhyde de n-butyle, et l'isomère du composé est un aldéhyde d'isobutyle :

[Formule 1]

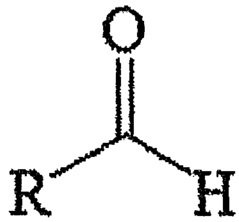

dans lequel R est un groupe alkyle en $C_1$ à $C_{12}$ ;

[Équation 1]

$$T_{t-2} - T_{b-3} \geq 8°C$$

[Équation 2]

$$P_2/P_1 \geq 20$$

dans lequel $T_{t-2}$ indique une température du flux supérieur de la deuxième colonne, et $T_{b-3}$ indique une température du flux inférieur de la troisième colonne, et
$P_1$ indique une pression de la section supérieure de la première colonne de distillation (kg/cm$^2$g), et
$P_2$ indique une pression de la section supérieure de la deuxième colonne de distillation (kg/cm$^2$g).

**15.** Procédé de préparation d'un composé représenté par la Formule 1, le procédé consistant à :

introduire une matière première comprenant le composé représenté par la Formule 1 ci-dessous et un isomère du composé dans un premier orifice d'alimentation d'une première colonne de distillation ;
décharger la matière première introduite vers chacun des flux supérieurs de la première colonne déchargés d'une section supérieure de la première colonne de distillation ; et des flux inférieurs des première, deuxième et troisième colonnes déchargés d'une section inférieure de la première colonne de distillation ;
introduire le flux inférieur de la première colonne dans un deuxième orifice d'alimentation d'une deuxième colonne de distillation,
décharger le flux introduit dans le deuxième orifice d'alimentation vers chacun des flux supérieurs de la deuxième colonne déchargés d'une section supérieure de la deuxième colonne de distillation ; et des flux inférieurs des quatrième et cinquième colonnes déchargés d'une section inférieure de la deuxième colonne de distillation ;

échanger la chaleur du flux supérieur de la deuxième colonne avec le flux inférieur de la troisième colonne ; et séparer le composé représenté par la Formule 1 de la section supérieure de la deuxième colonne de distillation, et l'isomère du composé représenté par la Formule 1 de la section supérieure de la première colonne de distillation,
dans lequel les Équations 1 et 2 ci-dessous sont satisfaites,
dans lequel le composé représenté par la Formule 1 est un aldéhyde de n-butyle, et l'isomère du composé est un aldéhyde d'isobutyle :

[Formule 1]

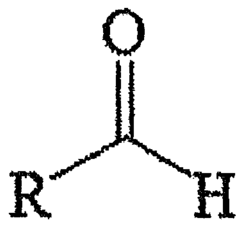

dans lequel R est un groupe alkyle en $C_1$ à $C_{12}$ ;

[Équation 1]

$$T_{t-2} - T_{b-3} \geq 8°C$$

[Équation 2]

$$P_2/P_1 \geq 20$$

dans lequel $T_{t-2}$ indique une température du flux supérieur de la deuxième colonne, et $T_{b-3}$ indique une température du flux inférieur de la troisième colonne, et
$P_1$ indique une pression de la section supérieure de la première colonne de distillation ($kg/cm^2g$), et
$P_2$ indique une pression de la section supérieure de la deuxième colonne de distillation ($kg/cm^2g$).

【Fig. 1】

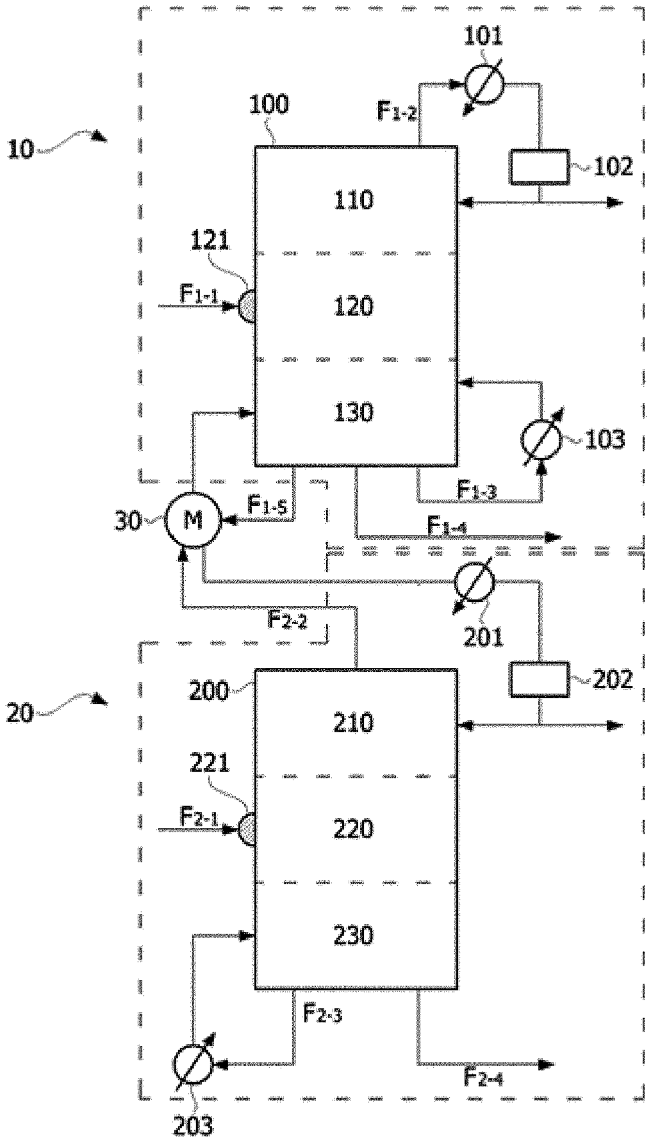
10
20
30
M
100
101
102
103
110
120
130
121
F1-1
F1-2
F1-3
F1-4
F1-5
200
201
202
203
210
220
230
221
F2-1
F2-2
F2-3
F2-4

【Fig. 2】

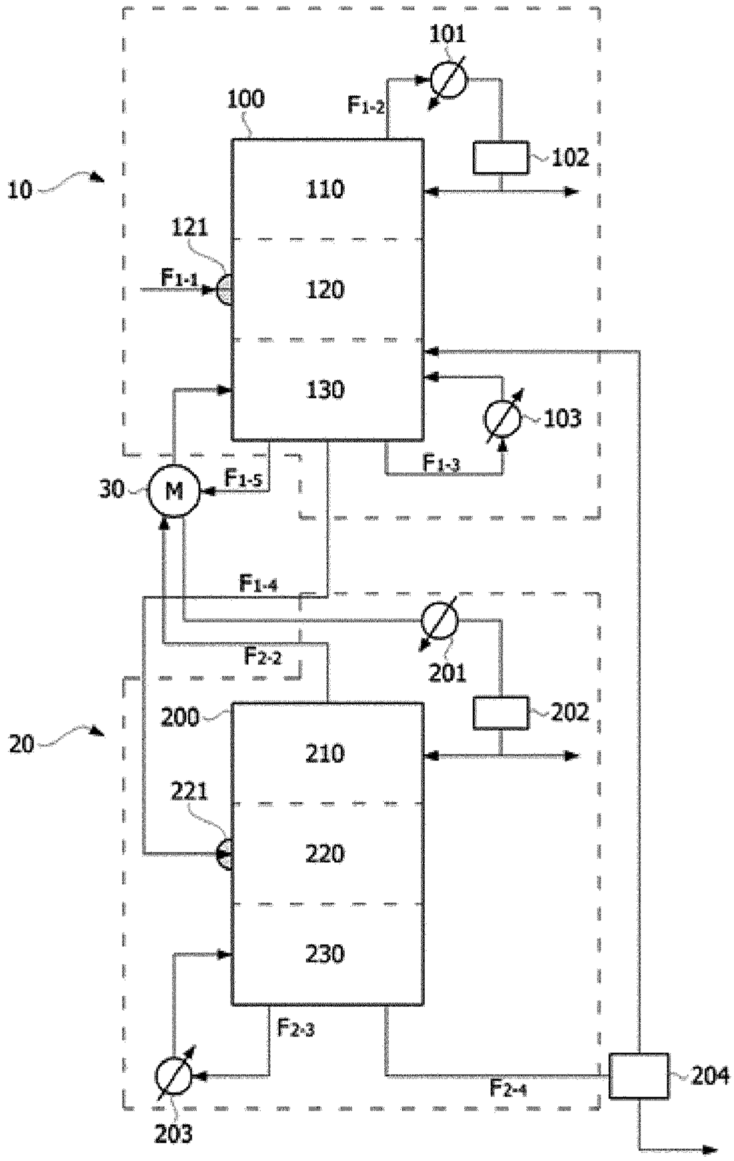

10
100
101
F1-2
102
110
121
F1-1
120
130
103
F1-3
30
M
F1-5
F1-4
201
F2-2
200
202
20
210
221
220
230
F2-3
203
F2-4
204

【Fig. 3】

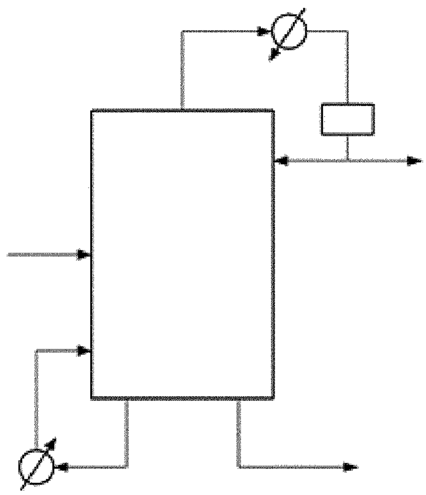

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 2015011651 W
- KR 1020140150416
- KR 1020150153090
- US 6511583 B
- RU 2130917 C1